# EUROPÄISCHE PATENTSCHRIFT

(11) **EP 0 978 508 B1**
(45) Veröffentlichungstag und Bekanntmachung des Hinweises auf die Patenterteilung: **14.12.2005**
(21) Anmeldenummer: 99114978.2
(22) Anmeldetag: 31.07.1999
(51) Int. Cl.: C07C 403/02, C07C 403/10, C07D 317/50, C07D 317/54, C07C 43/188

(54) **Herstellung von 4,4'-Diketo-carotinoiden**
Preparation of 4,4'-diketo-carotenoids
Préparation de 4,4'-dicéto-caroténoides

(30) Priorität: 05.08.1998 EP 98114684; 28.06.1999 EP 99112340
(43) Veröffentlichungstag der Anmeldung: 09.02.2000
(73) Patentinhaber: DSM IP Assets B.V., 6411 TE Heerlen (NL)
(72) Erfinder: Burdet, Bruno, 68390 Baldersheim (FR); Rüttimann, August, 4144 Arlesheim (CH)
(74) Vertreter: Mezger, Wolfgang

(56) Entgegenhaltungen:
- EP-A- 0 816 334
- M. MURAKAMI ET AL: CHEM. LETT., Nr. 6, 1987, Seiten 1051-1052, XP001109653
- M. ROSENBERGER ET AL: J. ORG. CHEM., Bd. 47, Nr. 11, 1982, Seiten 2130-2134, XP002230683

## Beschreibung

Die vorliegende Erfindung betrifft ein neues Verfahren zur Herstellung von symmetrischen, endständig ringsubstituierten Polyenen, insbesondere Carotinoiden der Typen Canthaxanthin, Astaxanthin usw. und den entsprechenden 2,2'-Dinor-carotinoiden (im allgemeinen als 4,4'-Diketo-carotinoide bezeichnet), aus acetalisierten Polyendialdehyden durch eine säurekatalysierte Kondensationsreaktion mit cyclischen Dienolethern.

Lewissäure-katalysierte Additionen von α,β-ungesättigten Ethern (Enolethern von Aldehyden) an Acetale sind schon lange bekannt und gehen auf die Arbeiten von Müller-Cunradi und Pieroh zurück (siehe US-Patentschrift 2.165.962). Hoaglin und Hirsch [J.A.C.S. 71, 3468 ff. (1949)] haben diese Reaktion weiter untersucht und die Anwendungsmöglichkeiten erweitert, was ebenfalls Isler et al. in den fünfziger Jahren getan haben, und zwar auf die Synthese von β-Carotin, Crocetindialdehyd, Lycopin sowie β-Apocarotinoiden [Helv. Chim. Acta 39, 249 ff. und 463 ff. (1956), ibid. 42, 854 ff. (1959) sowie US-Patentschriften 2.827.481 und 2.827.482]. Später erweiterte Mukaiyama die Reaktion durch Verwendung der gut und leicht zugänglichen Trimethylsilylenolether [Angew. Chem. 89, 858 ff. (1977) und Org. Reactions 28, 203 ff. (1982)].

Auch bei den Enolethern von aliphatischen und alicyclischen Ketonen reagieren sowohl Alkylenolether als auch Silylenolether mit Acetalen zu β-Alkoxy-ketonen oder unter Abspaltung von Alkohol zu den entsprechenden Eliminierungsprodukten [Chem. Lett. 1974, 16 ff. J.A.C.S. 102, 3248 ff. (1980), Chem. Lett. 1987, 1051 ff. sowie ibid., 1975, 569 ff.].

Ueber die ersten Lewissäure-katalysierten Kondensationen von 1-Alkoxy-1,3-dienen (Dienolethern) mit α,β-ungesättigten Acetalen wurde von Nazarov und Krasnaya [J. Gen. Chem. USSR 28, 2477 ff. (1958)] und von Makin [Pure & Appl. Chem. 47, 173 ff. (1976), J. Gen. Chem. USSR 31, 3096 ff. (1961) und 32, 3112 ff. (1962)] berichtet. Dabei erfolgt die Kopplung des Acetals an den Dienolether soweit ersichtlich ausschliesslich an dessen γ-Stellung unter Bildung eines kettenverlängerten α,β-ungesättigten Acetals, das jedoch in Konkurrenz zum ersten Acetal mit weiterem Dienolether reagiert unter Bildung eines weiteren, kettenverlängerten α,β-ungesättigten Acetals usw. [Telomerbildung; siehe auch noch Chemla et al., Bull. Soc. Chim. Fr. 130, 200 ff. (1993)]. Aus diesem Grund ist eine derartige Kondensation für synthetische Zwecke, speziell für die Synthese von Apocarotinalen, als nicht brauchbar befunden worden [Isler et al., Adv. Org. Chem. 4, 115 ff. (1963)].

Nicht nur 1-Alkoxy-1,3-diene, sondern auch Trimethylsilyloxydiene [des Typs CH₂=CH-CH=CH-OSi(CH₃)₃] können in Gegenwart von Lewissäure-Katalysatoren mit α,β-ungesättigten Acetalen kondensiert werden, wie dies Mukaiyama et al. in Chem. Lett. 1975, 319 ff. offenbarten. Auch bei dieser Kopplung scheint der Angriff ausschliesslich am endständigen (γ) Kohlenstoffatom des Diensystems zu erfolgen, ["γ-Angriff"; Mukaiyama et al., Bull. Chem. Soc. Japan 50, 1161 ff. (1977) sowie Japanische Patentpublikation (Kokai) 36.645/1977/Chem. Abs. 87, 201825 t (1977)]. Im Gegensatz zur Reaktion mit 1-Alkoxy-1,3-dienen, bei welcher ein α,β-ungesättigtes Acetal anfällt, wird bei der Umsetzung von Trimethylsilyloxydienen mit Acetalen ein Aldehyd gebildet, der mit dem Dien nicht weiterreagieren kann (keine Telomerbildung). Dabei werden als Katalysatoren Zinkbromid und viele andere Lewissäuren nur in kleinen Mengen benötigt [Fleming (et al.), Tetr. Lett. 1979, 3209 ff. und Chimia 34, 265 ff. (1980) sowie Brownbridge, Synth. 1983, 85 ff.]. Unter Verwendung dieser Methode konnten Mukaiyama et al. Vitamin A synthetisieren [Kokai 36.645/1977, Chem. Lett. 1975, 1201 ff. sowie Bull. Chem. Soc. Japan 51, 2077 ff. (1978)] und Mitarbeiter von Rhône-Poulenc neue Zugänge zu Carotinoiden und Vitamin A entwickeln (DOS 2.701.489 und A.E.C. Société de Chimie Organique et Biologique Nr. 7824350).

Die obenerwähnte, auf den Arbeiten von Nazarov und Krasnaya, Makin sowie Chemla et al. basierende Lewissäure-katalysierte Kondensation eines Dienolethers mit einem α,β-ungesättigten Acetal würde einen sehr wertvollen Zugang zu Apocarotinalen und Bis-apocarotinalen eröffnen, falls die Ausbeute am gewünschten Primärprodukt des Typs

...CH=CH-CH(OAlkyl¹)-CH₂-CH=CH-CH(OAlkyl¹)(OAlkyl²)

erhöht bzw. die Telomerbildung unterdrückt werden könnte. So konnte aus diesem Primärprodukt durch Hydrolyse der Acetalgruppe C(OAlkyl¹)(OAlkyl²) und Elimination von Alkyl¹OH der gewünschte Polyenaldehyd des Typs

...CH=CH-CH=CH-CH=CH-CHO

erhalten werden [Europäische Patentpublikation (EP) 0 816 334 A1].

Es sind einige Beispiele bekannt, bei denen Dienolether von Ketonen des Typs

...CH=CH-CH=C(O Alkyl/Trimethylsilyl)-CH₂-Akyl

mit Aldehyden, Acetalen, Orthoestern und anderen Elektrophilen zu α,β-ungesättigten Ketonen des Typs

...E-CH₂-CH=CH-CO-CH₂-Alkyl

(E stellt ein elektrophiles Substrat dar) umgesetzt werden [Tetr. Lett. 22, 705 ff. und 2833 ff. (1981), ibid., 27, 2703 ff. (1986), ibid. 29, 685 ff. (1988) sowie Chem Ber. 123, 1571 ff. (1990)]. Diese Reaktion scheint etwas beschränkt einsetzbar zu sein, nicht aus Reaktivitätsgründen, sondern wegen der schwierigen Zugänglichkeit der oben erwähnten Dienolether von Ketonen, da bei deren Herstellung u.a. mit Regioselektivitätsproblemen zu rechnen ist [Bildung der unerwünschten Regioisomeren des Typs

...CH₂-CH=CH-C(O-Alkyl/Trimethylsilyl)=CH-Alkyl].

Basierend auf der oben angesprochenen Dienoletherkondensation wurde von A. Rüttimann kürzlich eine neue, ergiebige Synthese von Apocarotinalen und Bis-apocarotinalen entwickelt (EP 0 816 334 A1), welche den Vorteil besitzt, dass die C-C-Verknüpfung unter katalytischen Bedingungen erfolgt, und zwar unter Verwendung eines Lewissäure-Katalysators. Zudem werden bei diesem Zugang keine phosphor- oder schwefelhaltigen Reagenzien benötigt.

Es wurde nun eine neue Synthese von Canthaxanthin, Astaxanthin, den entsprechenden 2,2'-Dinor-carotinoiden und strukturell ähnlichen, zwei endständige Ringe aufweisenden symmetrischen Carotinoiden (4,4'-Diketo-carotinoiden) gefunden. Diese neue Synthese basiert zwar ebenfalls auf einer katalysierten Dienoletherkondensation und vermeidet auch die Verwendung von phosphor- und schwefelhaltigen Reagenzien, macht allerdings auf ganz raffinierte und überraschende Weise Gebrauch von einer cyclischen Verbindung als Reaktionsteilnehmer, welche nicht nur die meisten Merkmale des endständigen Rings, sondern auch die für die Kondensation benötigte Dienolether-Gruppierung aufweist.

Ziel der vorliegenden Erfindung ist es, ausgehend von Polyendiacetalen die oben erwähnten symmetrischen Carotinoide herzustellen, und zwar unter möglichst weitgehender Vermeidung der obenerwähnten Nachteile des Standes der Technik sowie mit Ersatz der bisher zu diesem Zweck verwendeten Wittig-, Horner- oder Julia-Reaktion. Dieses Ziel wird erfindungsgemäss erreicht, indem man ein Polyendiacetal mit einem cyclischen Dienolether in Gegenwart eines geeigneten Katalysators, nämlich einer Lewis- oder Brönstedsäure, umsetzt und nach Hydrolyse des resultierenden Kondensationsproduktes eine basen- oder säureninduzierte Eliminierung von Alkohol an den beiden Enden der die beiden Ringe verbindenden, grösstenteils konjugierten Kohlenwasserstoffkette vornimmt, um das erwünschte symmetrische, endständig ringsubstituierte voll konjugierte Polyen zu erhalten. Nicht nur ist die Reaktion des cyclischen Dienolethers mit dem Polyendiacetal neu, sondern es erfolgt überraschenderweise dabei ein ausschliesslicher Angriff des Acetals an der γ-Stellung des Dienolethers. Durch die sich der Hydrolyse anschliessende, basen- oder säureninduzierte Elimination des Alkanols werden zwei konjugierte C-C-Doppelbindungen gebildet, ohne dass dazu ein phosphor- oder schwefelhaltiges Reagens benötigt wird, was im Gegensatz zu der bisher auf diesem Gebiet üblicherweise verwendeten Methodik steht.

Demnach handelt es sich bei der vorliegenden Erfindung um ein Verfahren zur Herstellung eines symmetrischen, endständig ringsubstituierten Polyens der allgemeinen Formel 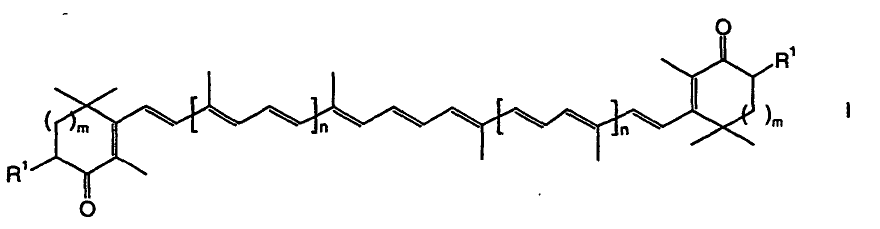 worin
- R¹: Wasserstoff oder Hydroxy,
- m: 0 oder 1 und
- n: 0, 1 oder 2 bedeuten,
das dadurch gekennzeichnet ist, dass man ein Polyen-di(O,O-dialkylacetal) der allgemeinen Formel 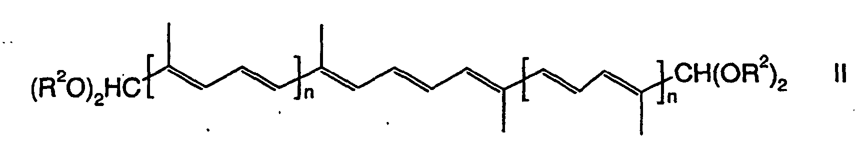 worin
- R²: C₁₋₆-Alkyl bedeutet und
- n: die oben angegebene Bedeutung besitzt,
mit einem cyclischen Dienolether der allgemeinen Formel 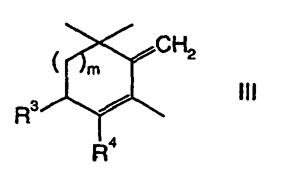 worin
- R³: Wasserstoff und
- R⁴: C₁₋₄-Alkoxy, oder
- R³ und R⁴: zusammen eine gegebenenfalls substituierte Methylendioxygruppe -O-C(R⁵)(R⁶)-O- und
- R⁵ und R⁶: unabhängig voneinander Wasserstoff, C₁₋₄-Alkyl oder Phenyl bedeuten,
- m: die oben angegebene Bedeutung besitzt,
in Gegenwart einer Lewis- oder Brönstedsäure umsetzt, das Reaktionsprodukt unter sauren Bedingungen hydrolysiert, und von der so erhaltenen Verbindung der allgemeinen Formel 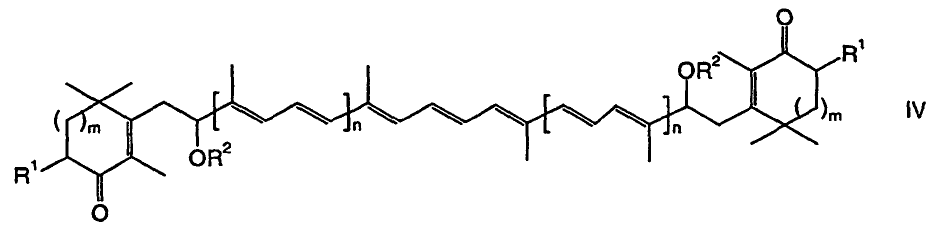 worin
- R¹: Wasserstoff oder Hydroxy bedeutet, je nachdem ob R³ und R⁴ der Formel III Wasserstoff resp. C₁₋₄-Alkoxy bzw. zusammen die gegebenenfalls substituierte Methylendioxygruppe bedeuten, und
- R², m und n: die oben angegebenen Bedeutungen besitzen,
unter basischen oder sauren Bedingungen das Alkanol R²OH abspaltet.

Im Rahmen der vorliegenden Erfindung umfasst der Ausdruck "C₁₋₄-Alkyl" bzw. "C₁₋₆-Alkyl" geradkettige und verzweigte Gruppen, wie beispielsweise Methyl, Ethyl, Isobutyl und Hexyl. Analoges gilt für den Alkylteil der Alkoxygruppe "C₁₋₄-Alkoxy".

Bei dem cyclischen Dienolether der Formel III handelt es sich um ein substituiertes Cyclopenten (m bedeutet 0, so dass die Formel III dann speziell die Formel 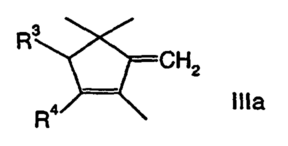 darstellt) oder ein substituiertes Cyclohexen (m bedeutet 1, so dass die Formel III in diesem Fall speziell die Formel 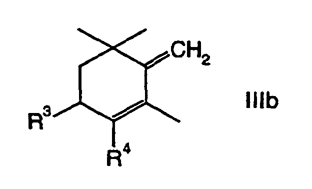 darstellt). In diesem Sinne sind unter den entsprechenden endständigen cyclischen Gruppen (Ringe), welche die Verbindungen der Formeln I und IV aufweisen, Gruppen der Formeln 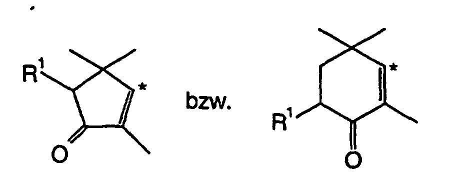 worin * die jeweilige Verknüpfungsstelle bezeichnet, zu verstehen.

Die im Rahmen der vorliegenden Erfindung offenbarten Formeln von Polyenen und cyclischen Dienolethern umfassen jeweils isomere Formen, z.B. optisch aktive und cis/trans bzw. E/Z-Isomere, sowie Gemische hiervon, sofern nicht ausdrücklich anders erwähnt. Was die E/Z-Isomerie anbelangt, so sind im allgemeinen die (all-E)-Isomeren der Polyen-di(O,O-dialkylacetale) der Formel II und der Produkte der Formel I des erfindungsgemässen Verfahrens bevorzugt.

Bei dem ersten Verfahrensschritt des erfindungsgemässen Verfahrens, d.h. der Umsetzung des Polyen-di(O,O-dialkylacetals) mit dem cyclischen Dienolether unter sauren Bedingungen, erfolgt ein ausschliesslicher Angriff der ersteren Verbindung an der γ-Stellung des cyclischen Dienolethers. Im Falle des Einsatzes eines cyclischen Dienolethers der Formel III, worin R³ Wasserstoff und R⁴ C₁₋₄-Alkoxy bedeuten, entsteht als Zwischenprodukt dieses ersten Verfahrensschrittes eine Verbindung der allgemeinen Formel 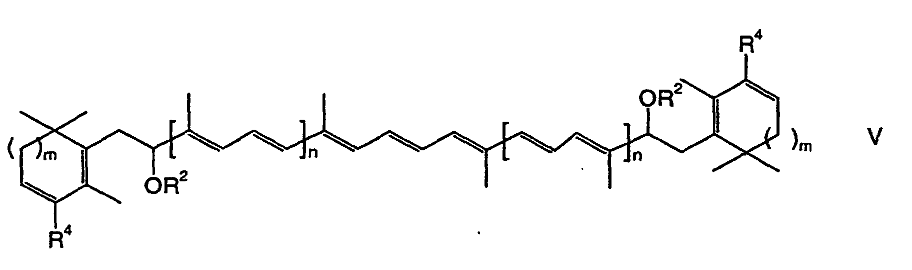 Im alternativen Fall, d.h. unter Einsatz eines cyclischen Dienolethers der Formel III, worin R³ und R⁴ zusammen die gegebenenfalls substituierte Methylendioxygruppe bedeuten, entsteht als Zwischenprodukt des ersten Verfahrensschrittes eine Verbindung der allgemeinen Formel

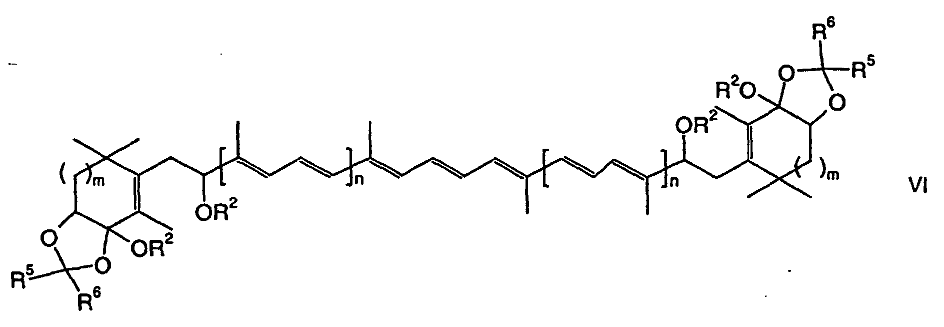

Dieser erste Verfahrenschritt wird zweckmässigerweise durchgeführt, indem man das Polyen-di(O,O-dialkylacetal) der Formel II mit dem cyclischen Dienolether der Formel III in einem organischen Lösungsmittel bei Temperaturen im Bereich von etwa -50°C bis etwa +60°C (z.B. von etwa -25°C bis etwa +60°C), vorzugsweise im Temperaturbereich von etwa -30°C bis zur Raumtemperatur (z.B. von etwa 0°C bis zur Raumtemperatur), und in Gegenwart einer Lewis- oder Brönstedsäure umsetzt. Als organische Lösungsmittel eignen sich im allgemeinen polare oder unpolare aprotische Lösungsmittel. Derartige Lösungsmittel sind beispielsweise niedere halogenierte aliphatische Kohlenwasserstoffe, z.B. Methylenchlorid und Chloroform; niedere aliphatische und cyclische Ether, z.B. Diethylether, tert.Butylmethylether und Tetrahydrofuran; niedere aliphatische Nitrile, z.B. Acetonitril; niedere aliphatische Ester, z.B. Ethylacetat; sowie aromatische Kohlenwasserstoffe, z.B. Toluen. Das bevorzugte Lösungsmittel ist Acetonitril, gegebenenfalls in Kombination mit weiteren oben erwähnten Lösungsmitteln, insbesondere mit Ethylacetat oder Methylenchlorid. Falls ein Gemisch von Acetonitril mit Ethylacetat oder Methylenchlorid verwendet wird, beträgt das Volumenverhältnis Acetonitril zu Ethylacetat bzw. Methylenchlorid vorzugsweise etwa 1 : 1 bis etwa 4 : 1, ganz bevorzugt etwa 4 : 1. Beispiele der verwendbaren Lewissäuren sind Zinkchlorid, Zinkchloriddietherat, Zinkbromid, Zinkdi(trifluormethan-sulfonat), Titantetrachlorid, Zinntetrachlorid, Bortrifluoridetherat sowie Eisen(III)chlorid; und der verwendbaren Brönstedsäuren sind p-Toluensulfonsäure, Methansulfonsäure, Trifluormethansulfonsäure, Schwefelsäure sowie Trifluoressigsäure. Im allgemeinen sind die Lewissäuren bevorzugt, insbesondere die Zinksalze, Bortrifluoridetherat und Eisen (III)chlorid. Die Katalysatoren werden im allgemeinen in katalytischen (unterstöchiometrischen) Mengen eingesetzt, und zwar zweckmässigerweise in einer Menge, die etwa 0,5 bis etwa 30 Molprozent bezogen auf die eingesetzte Menge des Polyen-di(O,O-dialkylacetals) beträgt, und vorzugsweise im Molprozentbereich von etwa 5% bis 10% liegt. Zudem verwendet man zweckmässigerweise etwa 2,1 bis etwa 4 Aequivalente cyclischen Dienolether pro Aequivalent Polyen-di(O,O-dialkylacetal), vorzugsweise etwa 2,2 bis etwa 2,6 Aequivalente. Ausserdem erfolgt die Umsetzung zweckmässigerweise bei Normaldruck, wobei im allgemeinen der Druck nicht kritisch ist.

Oftmals fällt das Zwischenprodukt der Formel V bzw. der Formel VI - in der Regel zusammen mit diversen ähnlichen Zwischenprodukten - als Niederschlag aus, welcher nach Abkühlung des Reaktionsgemisches, beispielsweise auf etwa -10°C bis -20°C, und Filtration isoliert werden kann. Anschliessend wird dann das Zwischenprodukt mit wässriger Säure zur entsprechenden Verbindung der Formel IV hyrolysiert.

Im Falle, dass keine Isolierung und anschliessende Hydrolyse vorgenommen wird, kann eine unmittelbare Hydrolyse im Reaktionsgemisch vorgenommen werden. Dabei gibt man zum Reaktionsgemisch eine Säure, vorzugsweise leicht verdünnte wässrige Essigsäure, beispielsweise mit einem Volumenverhältnis Essigsäure:Wasser von etwa 9:1, und rührt das Gemisch anschliessend eine Weile, beispielsweise etwa 30 Minuten bis etwa 2 Stunden, zweckmässigerweise im Temperaturbereich von etwa 0°C bis etwa 50°C. Zusätzlich zur Essigsäure kann man insbesondere eine katalytische Menge, wie etwa 1- 2 Molprozent bezogen auf die eingesetzte Menge des Polyen-di(O,O-dialkylacetals), von p-Toluensulfonsäure verwenden, um die Hydrolyse etwas zu beschleunigen. Gegenüber der separaten Hydrolyse des Zwischenproduktes der Formel V bzw. VI ist die im Reaktionsgemisch unmittelbar vorgenommene Hydrolyse bevorzugt.

Das Produkt der Formel IV kann in an sich bekannter Weise vom Reaktionsgemisch isoliert und gewünschtenfalls gereinigt werden. Typischerweise wird das Gemisch mit Wasser vereinigt und das Ganze mit einem mit Wasser nicht mischbaren organischen Lösungsmittel, wie beispielsweise mit einem niederen Alkan, Dialkylether oder aliphatischen Ester, z.B. Hexan, tert.Butyl-methylether bzw. Ethylacetat, extrahiert und die organische Phase mit Wasser und/oder Natriumbicarbonat- und/oder gesättigter wässriger Natriumchlorid-Lösung gewaschen, getrocknet und eingeengt. Das so isolierte und zumindest einigermassen gewaschene Rohprodukt kann dann gewünschtenfalls weitergereinigt werden, beispielsweise durch Säulenchromatographie, z.B. unter Verwendung von solchen Eluierungsmitteln als Hexan, Ethylacetat, Toluen oder Gemischen davon, oder (Um)Kristallisation, beispielsweise aus einem Alkohol, z.B. Methanol oder Ethanol.

Was den letzten Verfahrensschritt, d.h. die Abspaltung des Alkanols R²OH von der Verbindung der Formel IV, anbelangt, so sind Eliminierungen des Alkanols aus β-Alkoxyaldehyden oder δ-Alkoxy-α,β-ungesättigten Aldehyden unter Bildung der entsprechenden α,β-ungesättigten Aldehyde in der Fachliteratur bekannt und können unter verschiedenen Bedingungen durchgeführt werden. Beispielsweise wird im Rahmen bekannter baseninduzierter Eliminierungen als Base sehr oft 1,8-Diazabicydo[5.4.0]-undec-7-en verwendet, und zwar in einer Menge von etwa 2 bis 4 Aequivalenten bezogen auf die Menge eingesetzten Aldehyds. Es werden solche Bedingungen bei der bekannten Herstellung von Carotinoiden [siehe u.a. Bull. Chem. Soc. Japan 50, 1161 ff. (1977), ibid. 51, 2077 ff. (1978), Chem. Lett. 1975, 1201 ff. und Deutsche Offenlegungsschrift 2.701.489] und von Vitamin A (siehe u.a. Chem. Lett. 1975, 1201 ff.) verwendet. Als Beispiele von säureinduzierten Alkanolabspaltungen wird nochmals auf Bull. Chem. Soc. Japan 50, 1161 ff. (1977) sowie auf J. Gen. Chem. USSR 30, 3875 ff. (1960) verwiesen, bei denen p-Toluensulfonsäure bzw. 85%ige Phosphorsäure als saurer Katalysator verwendet wird. Besonders bei der Herstellung von Carotinoiden ist für eine derartige Abspaltung das Puffersystem Natriumacetat/Essigsäure [Helv. Chem. Acta. 39, 249 ff. und 463 ff. (1956) und US-Patentschriften 2.827.481 und 2.827.482] eingesetzt worden. Auch bei entsprechenden Alkoxyketonen (β-Alkoxy-ketonen oder δ-Alkoxy-α,β-ungesättigten Ketonen) gelingt die Abspaltung des Alkanols im allgemeinen sehr gut: siehe diesbezüglich Synthesis 1986, 1004 ff. bzw. J. Org. Chem. 49, 3604 ff. (1984). Unter Heranziehung dieser und anderer einschlägiger Literatur ist der Fachmann ohne weiteres in der Lage, geeignete Reaktionsbedingungen für die erfolgreiche Durchführung des letzten Schrittes des erfindungsgemässen Verfahrens zu finden.

Darüber hinaus kann die Abspaltung des Alkanols R²OH (2 Aequivalente pro Aequivalent der Verbindung der Formel IV) auch mit mehreren äquivalenten Mengen einer Base bezogen auf ein Aequivalent der Verbindung der Formel IV, durchgeführt werden. So wird der letzte Verfahrensschritt in diesem Fall zweckmässigerweise durchgeführt, indem man die in einem geeigneten organischen Lösungsmittel gelöste Verbindung der Formel IV in Gegenwart einer Base unter Abspaltung des Alkanols R²OH zum entsprechenden Polyen der Formel I umsetzt. Als organische Lösungsmittel eignen sich im allgemeinen protische, aprotische oder Gemische davon, wie beispielsweise Alkohole, z.B. Ethanol und Isopropanol, und Alkoholgemische; bzw. aromatische Kohlenwasserstoffe, z.B. Toluen. Die Base kann anorganisch oder organisch sein, und es eignen sich im allgemeinen starke Basen, insbesondere diejenigen Alkalimetallalkoholate, die stärkere Basen darstellen, z.B. Natriumethylat. Wie oben angedeutet, verwendet man zweckmässigerweise mindestens zwei Aequivalente Base pro Aequivalent der Verbindung der Formel IV, vorzugsweise etwa 2,5 bis etwa 8 Aequivalente.

Dabei wird zweckmässigerweise im Falle der Verwendung eines Alkalimetallalkoholates als Base im voraus entweder eine Lösung des Natriumalkoxids im Alkanol bereitgestellt, oder man stellt diese Lösung frisch aus metallischem Natrium und dem Alkanol her. Das Zusammenbringen der alkanolischen Lösung des Natriumalkoxids mit der vorzugsweise ebenfalls im voraus vorbereiteten Lösung oder Suspension der Verbindung der Formel IV in (dem gleichen) Alkanol kann in beliebiger Reihenfolge erfolgen. Man rührt dann das Reaktionsgemisch unter Erhitzen, geeigneterweise im Temperaturbereich von etwa 60°C bis etwa 140°C, vorzugsweise bei Temperaturen von etwa 80°C bis etwa 100°C. Die Umsetzung erfolgt je nach Siedepunkt des Lösungsmittels zweckmässigerweise bei Normaldruck oder bei leichtem Ueberdruck (um die gewünschte Temperatur zu erreichen), wobei im allgemeinen der Druck nicht kritisch ist. Unter diesen Bedingungen ist die Abspaltungsreaktion normalerweise nach einigen Stunden, insbesondere nach etwa 5 bis 10 Stunden, beendet.

Im Falle einer säureninduzierten Alkanolabspaltung eignen sich als Säuren im allgemeinen starke Mineralsäuren, wie beispielsweise Salzsäure, Bromwasserstoffsäure, Jodwasserstoffsäure, Schwefelsäure und Perchlorsäure, und Sulfonsäuren, wie beispielsweise Methansulfonsäure, Trifluormethansulfonsäure und p-Toluensulfonsäure. Die Mineralsäuren können wässrig sein, und zwar je nach Säure eine Konzentration von etwa 10 bis etwa 50 % aufweisen. Am besten geeignet ist Salzsäure (insbesondere etwa 10 bis 37%ige), Bromwasserstoffsäure (insbesondere etwa 25 bis 65%ige) oder Jodwasserstoffsäure (z.B. 47%ige). Erforderlich ist dabei nur eine katalytische Menge, d.h. bis höchstens 1 Aequivalent pro Aequivalent der Verbindung der Formel IV, vorzugsweise etwa 0,1 bis etwa 1 Aequivalent. Ferner kann die säureninduzierte Alkanolabspaltung in einem Lösungsmittel erfolgen, in welchem sich die Verbindung der Formel IV gut löst (eine sogenannte "homogene Abspaltung") oder in einem, in welchem dies nicht der Fall ist, d.h. in welchem sich die Verbindung der Formel IV eher in Suspension befindet (heterogene Abspaltung). In beiden Fällen muss sich allerdings der saure Katalysator nicht unbedingt lösen. Für die homogene Abspaltung geeignete Lösungsmittel sind insbesondere halogenierte aliphatische Kohlenwasserstoffe, z.B. Methylenchlorid, Chloroform und 1,2-Dichlorethan, und aromatische Kohlenwasserstoffe, z.B. Benzen und Toluen. Als Lösungsmittel (Dispersionsmittel) für die heterogene Abspaltung eignen sich niedere aliphatische Nitrile, Ketone und Carbonsäuren, z.B. Acetonitril, Aceton bzw. Essigsäure, vorzugsweise Acetonitril und Aceton. In beiden Fällen erfolgt die Alkanolabspaltung zweckmässigerweise im Temperaturbereich von etwa -20°C bis etwa +50°C, vorzugsweise im Bereich von etwa 0°C bis zur Raumtemperatur. Die Reaktionszeit ist jeweils abhängig von der Reaktionstemperatur und kann einige Stunden betragen, wobei die Abspaltungsreaktion normalerweise spätestens nach etwa 5 Stunden beendet ist.

Eine solche säureninduzierte Alkanolabspaltung eignet sich eher als eine baseninduzierte Alkanolabspaltung für die Herstellung von Astaxanthin, d.h. der Verbindung der Formel I, worin R¹ Hydroxy und m und n beide l bedeuten.

Als Alternative zur separaten Hydrolyse und Alkanol-Abspaltung können gewünschtenfalls mit einer etwas stärkeren Säure, insbesondere einer Mineralsäure, wie beispielsweise wässriger Salzsäure, diese beiden Verfahrensschritte in einem kombinierten Verfahrensschritt durchgeführt werden, ohne dass die Verbindung der Formel IV isoliert wird.

Ungeachtet der gewählten Prozedur des letzten Verfahrensschrittes kann das Produkt in an sich bekannter Weise vom Reaktionsgemisch isoliert werden, und zwar normalerweise durch Abkühlung des Reaktionsgemisches, zweckmässigerweise auf Raumtemperatur oder sogar bis auf etwa 0°C, gegebenenfalls Zugabe von Wasser und Abfiltration. Nach dessen Isolierung kann das Produkt gewaschen, beispielsweise mit Wasser und/oder wässrigem Alkohol, und schliesslich gegebenenfalls unter vermindertem Druck getrocknet werden. Gewünschtenfalls können solche weitere Methoden wie Säulenchromatographie und Umkristallisation eingesetzt werden, um zu einem noch reineren Produkt zu gelangen. Falls eine Isomerisierung der im Produkt vorhandenen Z-Isomeren zu den entsprechenden E-Isomeren erwünscht wird, kann im Isolierungs- und Reinigungsverfahren eine diesbezügliche Zwischenstufe eingeschaltet werden; diese besteht darin, dass man unmittelbar nach der Abkühlung einen Alkohol oder wässrigen Alkohol, z.B. wässriges Isopropanol, zugibt, das Gemisch im Temperaturbereich von etwa 80°C bis etwa 100°C erhitzt, und danach das Gemisch wieder abkühlt und den Festkörper abfiltriert und trocknet. Als mögliche Lösungsmittel kommen auch noch gesättigte niedrige Kohlenwasserstoffe, z.B. Heptan, in Frage. Im allgemeinen sind die E-Isomeren weniger löslich als die entsprechenden Z-Isomeren und fallen demzufolge oft als Niederschlag in grösserer Ausbeute aus. Darüber hinaus - wie oben erwähnt - sind im allgemeinen die (allE)-Isomeren der Produkte der Formel I bevorzugt.

In dem oben definierten erfindungsgemässen Verfahren bedeuten R² vorzugsweise Methyl, entweder R³ vorzugsweise Wasserstoff und R⁴ vorzugsweise Isobutoxy oder R³ und R⁴ zusammen vorzugsweise die Methylendioxygruppe (R⁵ = R⁶ = Wasserstoff), und n vorzugsweise 1.

Während einige der Edukte des erfindungsgemässen Verfahrens bekannt sind, sind andere aus zum Teil bekannten Vorstufen nach an sich bekannten Methoden herstellbar.

So können beispielsweise die neuen Polyen-di(O,O-dialkylacetale) der Formel II sehr einfach auf bekannte allgemeine Weise durch Umsetzung des entsprechenden Polyendialdehyds der Formel 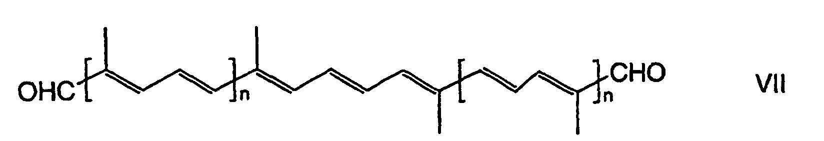 mit dem diesbezüglichen Orthoameisensäure-trialkylester hergestellt werden, insbesondere in dem entsprechenden C₁₋₆-Alkanol, z.B. Methanol für das O,O-Dimethylacetal, und in Gegenwart einer katalytischen Menge einer organischen Säure oder einer Lewissäure, z.B. p-Toluensulfonsäure bzw. Zinkchlorid [siehe beispielsweise Organikum, Organischchemisches Grundpraktikum, 6. Auflage, S. 377 ff. (1963)]. Die Reaktion verläuft zum Teil in Suspension, d.h. der jeweilige Polyen-dialdehyd wird im Alkanol oder in einem Alkanol/Methylenchlorid-Gemisch suspendiert, und dann werden zweckmässigerweise etwa vier Moläquivalente des Orthoameisensäure-trialkylesters zur Suspension gegeben, gefolgt von einer Spur sauren Katalysators, z.B. p-Toluensulfonsäure. Dabei löst sich der Dialdehyd langsam auf, und das gebildete Polyen-di(O,O-dialkylacetal) der Formel II kristallisiert gleichzeitig langsam aus. Die Umsetzung wird zweckmässigerweise im Temperaturbereich von etwa 0°C bis etwa 40°C durchgeführt und dauert in der Regel von etwa 30 Minuten bis etwa 4 Stunden. Als weitere Literaturstellen, welche die allgemein bekannte Acetalisierungsmethode veranschaulichen, wird auf die europäischen Patentpublikationen 252 389 und 391 033 sowie auf J. Mol. Cat. 79, 117 ff. (1993) verwiesen.

Die Polyen-dialdehyde der Formel VII ihrerseits sind entweder bekannt, insbesondere aus der Fachliteratur betreffend Carotinoide, oder - falls neu - können nach an sich bekannten einschägigen Methoden hergestellt werden. So sind beispielsweise aus dieser Literatur die zweifache Umsetzung des 2,7-Dimethyl-2,4,6-octatrien-1,8-dials (des sogenannten "C₁₀-Dials") mit C₅- oder C₁₀-Wittigaldehyden zu verschiedenen kettenverlängerten Dialdehyden bekannt geworden. Die Lehrbücher "Carotinoids" (O. Isler, Birkhäuser Verlag Basel und Stuttgart, 1971), besonders dessen Kapitel VI und XII und die darin erwähnte weitere Literatur, und "Carotenoids, Volume 2 : Synthesis" (G. Britton, S. Liaaen-Jensen und H. Pfander, Birkhäuser Verlag Basel Boston Berlin, 1996) besonders dessen Kapitel III und VII, liefern viele nützliche Hinweise auf die Herstellung und das Vorkommen der bekannten Dialdehyde.

Die cyclischen Dienolether der Formel III sind neu und stellen einen weiteren Aspekt der vorliegenden Erfindung dar.

Diejenigen cyclischen Dienolether der Formel III, worin R³ Wasserstoff, R⁴ C₁₋₄-Alkoxy und m 0 bedeuten, können gemäss dem nachfolgenden Reaktionsschema 1 hergestellt werden, und zwar ausgehend von dem bekannten 2-Methyl-1,3-cyclopentan-dion:

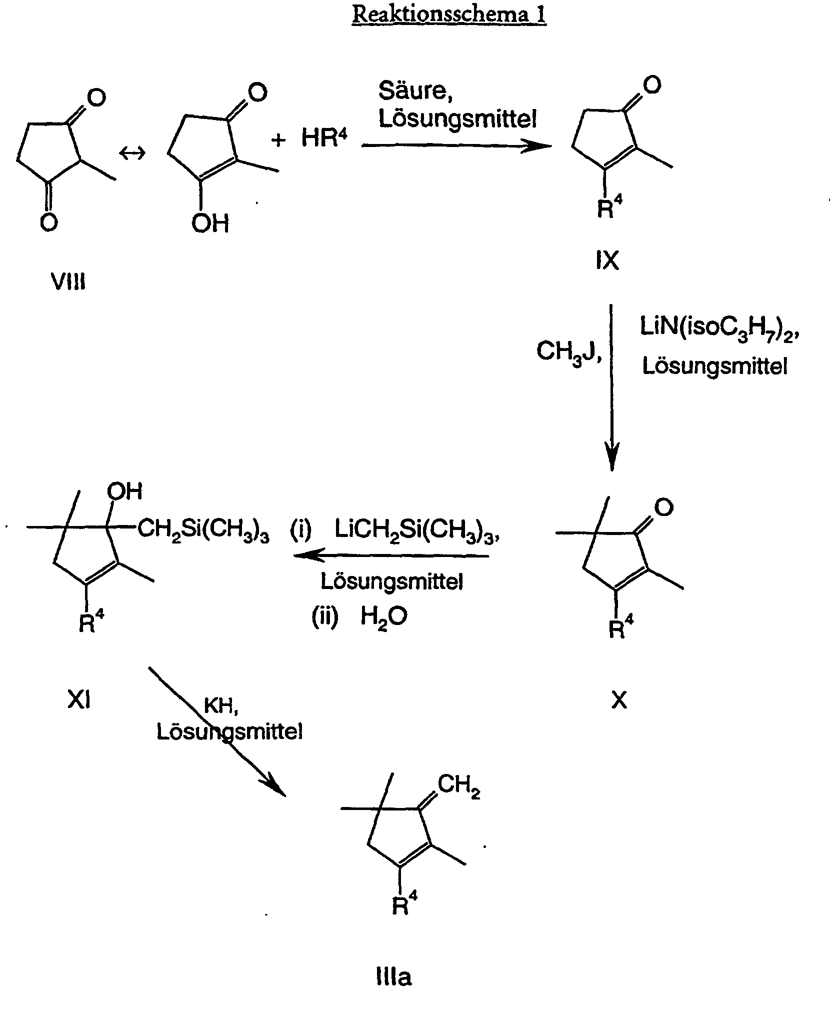

Diejenigen Verbindungen der Formel X, worin R⁴ Methoxy oder Isobutoxy bedeutet, sind bekannt und lassen sich nach der Methode von Rosenberger et al. [J. Org. Chem. 47, 2134 ff. (1982)] aus dem käuflichen 2-Methyl-1,3-cydopentandion/1-Hydroxy-2-methylcyclopenten-3-on der Formel VIII durch säurekatalysierte Veretherung mit Methanol bzw. Isobutanol zur entsprechenden Verbindung der Formel IX, gefolgt von einer doppelten Methylierung mit Methyljodid und Lithiumdiisopropylamin bei niedriger Temperatur, z.B. bei etwa -70°C, herstellen. Als saurer Katalysator/Lösungsmittel-Kombination für den ersten Verfahrensschritt VIII → IX eignet sich insbesondere p-Toluensulfonsäure/Toluen, und für den zweiten Verfahrensschritt verwendet man als Lösungsmittel vorzugsweise Tetrahydrofuran. Die restlichen Verbindungen der Formel X, worin R⁴ eine andere C₁₋₄-Alkoxygruppe als Methoxy oder Isobutoxy bedeutet, lassen sich auf analoge Weise herstellen.

Gemäss der Peterson-Olefinierung [J. Org. Chem. 33, 780 ff. (1968]) wird dann der Ketoenolether der Formel X mit Trimethylsilylmethylithium (geeigneterweise selber aus Trimethylsilylmethylchlorid und metallischem Lithium in Pentan hergestellt) in Pentan umgesetzt, was nach anschliessender Zugabe von Wasser die Verbindung der Formel XI in kristalliner Form ergibt. Anschliessend kann diese unmittelbar mit Kaliumhydrid als Base und in Tetrahydrofuran als Lösungsmittel bei Temperaturen unter der Raumtemperatur, z.B. im Temperaturbereich von etwa 0°C bis etwa 15°C, zum gewünschten cyclischen Dienolether der Formel IIIa umgesetzt werden. Dabei wird zweckmässigerweise das als Lösungsmittel im Verfahrensschritt X → XI verwendete Pentan destillativ durch das Lösungsmittel des letzten Verfahrensschrittes XI → IIIa Tetrahydrofuran ersetzt, bis ein Siedepunkt von etwa 62°C (Siedepunkt von Tetrahydrofuran 66°C) erreicht wird. Es ist nicht notwendig, die intermediär erzeugte Verbindung der Formel XI zu isolieren: durch den Lösungsmittelaustausch und die thermische Behandlung zerfällt diese Verbindung in den gewünschten cyclischen Dienolether der Formel IIIa und das Lithiumsalz von Trimethylsilanol.

Nach Zugabe von Wasser wird der so erhaltene Dienolether zweckmässigerweise mit einem geeigneten Lösungsmittel, insbesondre einem niederen Alkan, z.B. Pentan oder Hexan, oder einem niederen aliphatischen Ether, z.B. Diethylether, extrahiert und danach unter Hochvakuum destillativ gereinigt.

Diejenigen cyclischen Dienolether der Formel III, worin R³ Wasserstoff, R⁴ C₁₋₄-Alkoxy und m 1 bedeuten, können gemäss dem nachfolgenden Reaktionsschema 2 hergestellt werden:

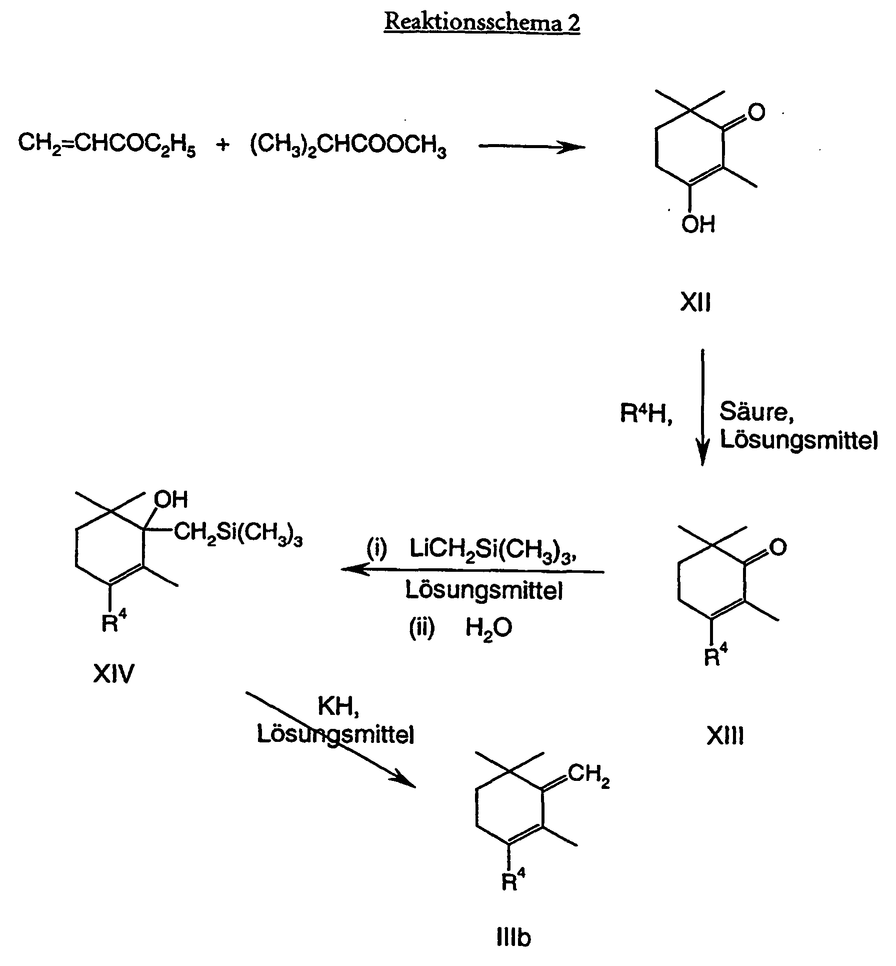

Diejenigen Verbindungen der Formel XIII, worin R⁴ Methoxy, Ethoxy oder Isobutoxy bedeutet, sind bekannt [Tetr. Lett. 37, 1015 ff. (1996) bzw. EP 31875] und lassen sich nach der Methode von Rosenberger et al. J. Org. Chem. 47, 2130 (1982) aus Isobuttersäuremethylester und Ethylvinylketon (durch eine Robinson-Annellierung) gefolgt von einer säurekatalysierten Veretherung des resultierenden 1-Hydroxycyclohexen-3-ons der Formel XII mit dem entsprechenden Alkanol zur entsprechenden Verbindung der Formel XIII herstellen. Als saurer Katalysator für den letzteren Verfahrensschritt XII → XIII eignet sich insbesondere Methansulfonsäure oder p-Toluensulfonsäure und als Lösungsmittel insbesondere ein niederes Alkan, z.B. Hexan, oder ein aromatischer Kohlenwasserstoff, z.B. Benzen oder Toluen. Die restlichen Verbindungen der Formel XIII, worin R⁴ eine andere C₁₋₄-Alkoxygruppe als Methoxy, Ethoxy oder Isobutoxy bedeutet, lassen sich auf analoge Weise herstellen.

Der dritte und der letzte Verfahrensschritt zum gewünschten cyclischen Dienolether der Formel IIIb können analog dem Verfahrensschritt X → XI und dem Verfahrensschritt XI → IIIa der Herstellung der entsprechenden Fünfring-Verbindung durchgeführt werden [gemäss der Peterson-Olefinierung, J. Org. Chem 33, 780 ff. (1980)]. Obwohl die Verbindung der Formel XIV isoliert und durch Kristallisation gereinigt werden kann, ist sie jedoch - besonders in reiner kristalliner Form - sehr unstabil; sie lagert sich an der Luft leicht in die Verbindung der Formel 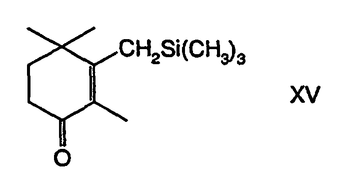 um. Daher muss die Verbindung der Formel XIV nach einer Kristallisation und Trocknung, geeigneterweise unter Hochvakuum und Begasung mit inertem Gas, z.B. Argon, sobald wie möglich im nächsten (letzten) Verfahrensschritt eingesetzt werden. Dieser letzte Verfahrensschritt erfolgt zweckmässigerweise in Gegenwart von Kaliumhydrid als Base und in Tetrahydrofuran als Lösungsmittel bei Temperaturen im Bereich von etwa 0°C bis etwa 15°C.

Nach Zugabe von Wasser wird der so erhaltene Dienolether zweckmässigerweise mit einem geeigneten Lösungsmittel, insbesondere einem niederen Alkan, z.B. Pentan oder Hexan, oder einem niederen aliphatischen Ether, z.B. Diethylether, extrahiert und danach unter Hochvakuum destillativ gereinigt.

Effizienter wird diese Herstellung des cyclischen Dienolethers der Formel IIIb durchgeführt, indem man die Verbindung der Formel XIII mit Trimethylsilylmethyllithium in Pentan bei Temperaturen von etwa 0°C bis etwa -10°C umsetzt und danach das Pentan durch Tetrahydrofuran ersetzt, bis der Siedepunkt des Tetrahydrofurans erreicht wird; es handelt sich dabei um ein Eintopfverfahren. Wie in der oben beschriebenen Herstellung des cyclischen Enolethers der Formel IIIa zerfällt dabei das entstandene Zwischenprodukt der Formel XIV in den gewünschten cyclischen Enolether der Formel IIIb und das Lithiumsalz von Trimethylsilanol. Zu langes Erhitzen in Tetrahydrofuran sollte allerdings vermieden werden, da unter den herrschenden basischen Bedingungen eine teilweise Isomerisierung des entstandenen cyclischen Enolethers in das entsprechende Cyclohexadien der Formel 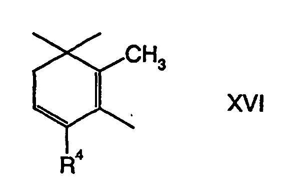 erfolgen kann.

Schliesslich können diejenigen cyclischen Dienolether der Formel III, worin R³ und R⁴ zusammen eine gegebenenfalls substituierte Methylendioxygruppe -O-C(R⁵)(R⁶)-O-bedeuten, gemäss dem nachfolgenden Reaktionsschema 3 hergestellt werden, und zwar ausgehend von dem bekannten 1,5-Dihydroxy-2,4,4-trimethyl-cyclopent-1-en-3-on bzw. 1,6-Dihydroxy-2,4,4-trimethyl-cyclohex-1-en-3-on:

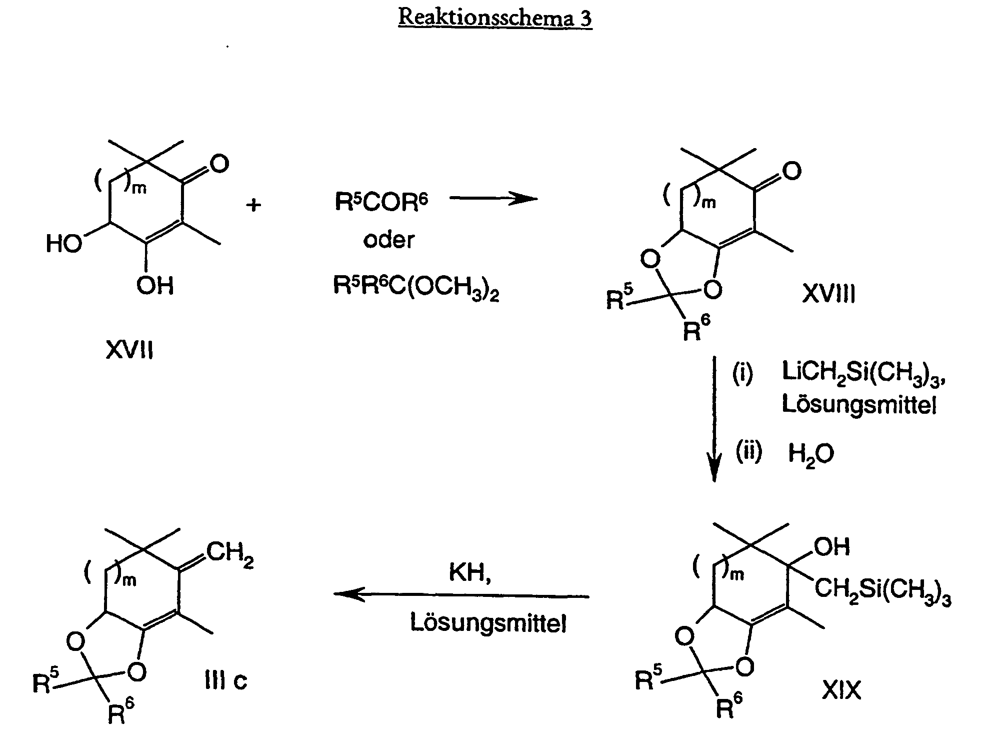

Die Verbindung der Formel XVII wird auf an sich bekannte Weise mit dem Keton R⁵COR⁶ oder dessen Dimethylacetal zur entsprechenden Verbindung der Formel XVIII acetalisiert [siehe Helv. Chim. Acta 64, 2436 ff. (1981) und EP 0 085 158 A2]. Falls als Keton oder Dimethylacetal Aceton bzw. dessen Dimethylacetal verwendet wird, ist in diesem Fall die so erhaltene Verbindung der Formel XVIII, worin R⁵ und R⁶ beide Methyl und m 0 oder 1 bedeuten, bekannt.Vorzugsweise verwendet man allerdings Formaldehyd oder Formaldehyd-dimethylacetal als Acetalisierungsreagens und erhält so die Verbindung der Formel XVIII, worin R⁵ und R⁶ beide Wasserstoff bedeuten, in hoher Ausbeute. Die nächsten beiden Verfahrensschritte XVIII → XIX und XIX → IIIc können analog den Verfahrensschritten X → XI und XI → IIIa oder XIII → XIV und XIV → IIIb des Reaktionsschemas 1 bzw. 2 durchgeführt werden. Das bevorzugte Produkt dieses Verfahrens ist 4,6,6-Trimethyl-5,6,7,7a-tetrahydro-5-methyliden-1,3-benzodioxol, d.h. die Verbindung der Formel IIIc, worin R⁵ und R⁶ beide Wasserstoff und m 1 bedeuten.

Nicht nur die cyclischen Dienolether der Formel III, sondern auch die Zwischenprodukte der Formeln IV, V und VI sind neue Verbindungen und stellen ebenfalls einen weiteren Aspekt der vorliegenden Erfindung dar.

Die Endprodukte des erfindungsgemässen Verfahrens, d.h. die symmetrischen, endständig ringsubstituierten Polyene der allgemeinen Formel I, gehören grösstenteils dem Gebiet der Carotinoide an und finden entsprechende Verwendung, beispielsweise als Farbstoffe bzw. Pigmente für Lebensmittel, den Eidotter, die Integumente (insbesondere Haut, Ständer und Schnäbel) und/oder das subkutane Fett von Geflügel, das Fleisch und/oder die Integumente (insbesondere Haut, Schuppen und Schale) von Fischen und Crustaceen usw. Beispielsweise eignet sich Astaxanthin hervorragend als Pigmenter bei der Lachspigmentierung. Diese Verwendung kann nach an sich bekannten Methoden erfolgen, wie diese beispielsweise in der europäischen Patentpublikation Nr. 630.578 beschrieben ist.

Die Erfindung wird anhand der nachfolgenden Beispiele veranschaulicht:

### A. Herstellung der Polyen-di(O,O-dialkylacetale) (Verbindungen der Formel II)

### Beispiel 1

### 8,8'-Diapocarotinal-dimethylacetal (Crocetindialdehyd-dimethylacetal)

In einem mit Magnetrührer und Argonbegasung ausgestatteten 500 ml-Rundkolben wurden 15g (50,1 mmol) Croctindialdehyd (nach HPLC ≥ 99% rein) und 30 g (141 mmol) Orthoameisensäure-trimethylester in 50ml Methylenchlorid und 40 ml Methanol suspendiert. Unter Rühren gab man bei Raumtemperatur 60 mg p-Toluensulfonsäure-Monohydrat zu. Die vorhandenen Kristalle lösten sich in etwa 2 - 3 Minuten auf, und nach weiteren etwa 5 Minuten bildete sich ein gelber Niederschlag. Nach etwa 40 minütigem Rühren gab man tropfenweise 250 ml Methanol, gefolgt von 0,3 ml Triethylamin zu. Es wurden anschliessend unter vermindertem Druck (350 - 400 mbar/35-40 kPa) bei 30°C etwa 50 ml Lösungsmittel, d.h. Methylenchlorid, während 30 Minuten abdestilliert. Dann wurde mittels eines Eisbades auf 0°C abgekühlt, abfiltriert, mit Methanol bei -10°C gewaschen und unter Hochvakuum bei Raumtemperatur getrocknet. Dies ergab 17,8 g Crocetindialdehyddimethylacetal als gelb-orange Kristalle. Umkristallisation aus 50 ml erwärmtem Methylenchlorid und 260 ml dazu getropftem Methanol unter Rühren und anschliessendes Abkühlen bis auf 0°C ergab nach Filtration, Waschen mit Methanol bei 10°C und Trocknung unter Hochvakuum bei Raumtemperatur 17,02 g (87%ige Ausbeute) Crocetindialdehyd-dimethylacetal als gelb-orange Kristalle, Smp. 138°C, mit einem Gehalt nach HPLC von > 99%; UV (Hexan/2% Methylenchlorid): 456 nm (logE = 4,63), 423 nm (logE = 5,02), 398 nm (logE = 4,90), 378 nm (logE = 4,63); ¹H-NMR (C₆D₆, 400 MHz): 1,83 (s, 6H), 1,87 (s, 6H), 3,18 (s, 12H), 4,59 (s, 2H), 6,25 - 6,70 (m, 10 olefinische H);

| Mikroanalyse: | | |
|---|---|---|
| Ber. | C 74,19% | H 9,34% |
| Gef. | C 74,01% | H 9,32% |

### Beispiel 2

### 8,8'-Diapocarotinal-diethylacetal (Crocetindialdehyd-diethylacetal)

In einem mit Magnetrührer und Argonbegasung ausgestatteten 250 ml-Rundkolben wurden bei Raumtemperatur 10,0 g (33,4 mmol) Crocetindialdehyd (nach HPLC ≥ 99% rein) in 30 ml Methylenchlorid und 70 ml Ethanol suspendiert. Unter Rühren gab man bei Raumtemperatur 22 g (140 mmol) Orthoameisensäure-triethylester und 50 mg p-Toluensulfonsäure-Monohydrat zu. Die vorhandenen Kristalle lösten sich in etwa 10 - 15 Minuten auf, und es bildete sich eine dunkelgrüne Lösung. Nach weiterem 15-minütigem Rühren bei Raumtemperatur wurde mit 0,5 ml Triethylamin neutralisiert und anschliessend unter vermindertem Druck (200 - 120 mbar/20 - 12 kPa) bei Raumtemperatur das Methylenchlorid entfernt. Dabei fielen orange Kristalle aus. Nun wurden weitere 30 ml Ethanol zugeben, auf 0°C abgekühlt, die Kristalle abfiltriert und mit Ethanol bei -10°C gewaschen. Dies ergab nach Trocknung unter Hochvakuum bei Raumtemperatur 11,23 g Crocetindialdehyd-diethylacetal als feines oranges Pulver, Smp. 128 - 129°C. Umkristallisation aus Methylenchlorid/Ethanol bei 0°C ergab 10,4 g (68%ige Ausbeute) Crocetindialdehyd-diethylacetal als feine, gelb-orange Kristalle, Smp. 130 - 130,5°C, mit einem Gehalt nach HPLC von 97,5%; UV (Cyclohexan/3% Methylenchlorid): 462 nm (logE = 5,11), 434 nm (logE = 5,10), 411 nm (logE = 4,88); Massenspektrum: 444 (M⁺, 80); ¹H-NMR (C₆D₆, 400 MHz): 1,26 (t, J = 7 Hz, 6H), 1,94 und 2,04 (2s, je 3H), 3,50 und 3,70 (2m, je 2H), 4,90 (s, 1H), 6,4 - 6,8 (5 olefinische H);

| Mikroanalyse: | | |
|---|---|---|
| Ber. | C 75,63% | H 9,97% |
| Gef. | C 75,44% | H 9,86% |

### B. Herstellung der cyclischen Dienolether (Verbindungen der Formel III)

### Beispiel 3

### 1-Isobutoxy-2,4,4-trimethyl-3-exomethylen-cyclopenten (Formel III, worin R³ Wasserstoff, R⁴ Isobutoxy und m 0 bedeuten)

### (i) Herstellung von 1-Isobutoxy-2,4,4-trimethyl-cyclopenten-3-on

Zur Herstellung einer Lösung von Lithiumdiisopropylamin in Tetrahydrofuran ("LDA-Lösung") tropfte man zu einer Mischung von 470 ml (750 mmol) einer 1,6 M-Lösung von Butyllithium in Hexan und 390 ml Tetrahydrofuran unter Argon innert 30 Minuten 117 ml (830 mmol) Diisopropylamin zu und liess die Temperatur langsam auf -15°Ckommen.

In einem mit mechanischem Rührer, Thermometer, 500 ml-Tropftrichter und Argonbegasung ausgestatteten 1,5l-Sulfierkolben wurden 430 ml (etwa 330 mmol) obiger LDA-Lösung bei -70°C vorgelegt. Dazu tropfte man 55 g (327 mmol) 1-Isobutoxy-2-methyl-cyclopenten-3-on. Man rührte dann das Reaktionsgemisch 20 Minuten bei -70°C, versetzte es anschliessend langsam mit 20,3 ml (46,3 g, 326 mmol) Methyljodid und liess es auf Raumtemperatur kommen. Nach 15-minütigem Rühren bei Raumtemperatur wurde erneut auf -70°C abgekühlt und weitere 300 ml (230 mmol) obiger LDA-Lösung tropfenweise zugegeben, gefolgt von 14,1 ml (32,1 g, 226 mmol) Methyljodid. Man liess erneut auf Raumtemperatur kommen und wiederholte diese Prozedur noch dreimal mit 128 ml (98 mmol) LDA-Lösung und 6,1 ml (14 g, 100 mmol) Methyljodid, 86 ml (66 mmol) LDA-Lösung und 4,0 ml (9 g, 65 mmol) Methyljodid sowie 42 ml (32 mmol) LDA-Lösung und 2,0 ml (4,5 g, 32 mmol) Methyljodid. Das Reaktionsgemisch wurde danach eine Stunde bei Raumtemperatur gerührt.

Zur Aufarbeitung gab man langsam, zuerst tropfenweise, 100 ml Wasser, dann 500 ml Diethylether zu, trennte die wässrige Phase ab und wusch die organische Phase mit etwa 500 ml gesättigter Natriumchlorid-Lösung. Die organische Phase wurde nun über wasserfreiem Natriumsulfat getrocknet und unter vermindertem Druck bei 35°C eingeengt. Dies ergab 65 g rohes 1-Isobutoxy-2,4,4-trimethyl-cyclopenten-3-on als farbloses Oel.

Diese Methylierungsreaktion wurde nochmals ganz analog wiederholt, und zwar ausgehend von 49 g (291 mmol)1-Isobutoxy-2-methyl-cyclopenten-3-on. Es wurden auf diese Weise weitere 49 g rohes 1-Isobutoxy-2,4,4-trimethyl-cyclopenten-3-on erhalten.

Die rohen Produkte (zusammen 114 g) dieser beiden Ansätze wurden durch Destillation über eine kurze Vigreux-Kolonne [Sdp. etwa 83°C/0,4 mbar (40 Pa)] gereinigt. Dies ergab 85 g Produkt, das teilweise glasartig erstarrte. Das Produkt wurde in 500 ml Pentan gelöst und im Tiefkühlschrank (-25°C) aus der Lösung kristallisiert. Nach Filtration wurden die Kristalle unter Vakuum (14 mmHg/1,85 kPa) bei Raumtemperatur getrocknet, was 79,6 g (66%ige Ausbeute) 1-Isobutoxy-2,4,4-trimethyl-cyclopenten-3-on als schneeweisse Blättchen, Smp. 63°C, ergab. Der Gehalt am gewünschten Produkt nach Gaschromatographie (GC) betrug 100%.

### (ii) Herstellung von 1-Isobutoxy-2,4,4-trimethyl-3-exomethylen-cyclopenten

In einem mit mechanischem Rührer, Thermometer, Tropftrichter und Argonbegasung ausgestatteten 500 ml-Vierhalssulfierkolben wurden 170 ml einer etwa 0,8 M-Lösung von Trimethylsilylmethyllithium (etwa 140 mmol) in Pentan [hergestellt durch etwa 16-stündiges Erhitzen von 3,3 g (0,48 mol) Lithium-Pulver in 140 ml Pentan mit 24,7 g (0,2 mol) Chlormethyltrimethylsilan bei 40°C (Rückflusstemperatur), Filtrieren unter Argon mit Drucknutsche und Spülen mit etwa 20 ml Pentan; Ausbeute jeweils etwa 80 - 85% gemäss Titration: siehe J. Organomet. Chem. 9, 165 -168 (1967)] vorgelegt. Bei - 20°C gab man dazu tropfenweise eine Lösung von 23 g (112 mmol) 1-Isobutoxy-2,4,4-trimethylcyclopenten-3-on (96%ig rein gemäss GC) in 50 ml Tetrahydrofuran. Dann wurde auf Raumtemperatur erwärmt, eine Vigreux-Kolonne aufgesetzt und Lösungsmittel (Pentan) abdestilliert und kontinuierliche Zugabe von Tetrahydrofuran (schliesslich 300 mol) vorgenommen, bis eine Kopftemperatur von 62°C erreicht wurde. Dann wurde auf 0°C abgekühlt und 150 ml Wasser zugetropft. Nach Extraktion mit Pentan und Waschen der organischen Phase mit gesättigter Natriumcarbonat- und Natriumchlorid-Lösung wurde die organische Phase über wasserfreiem Natriumsulfat getrocknet, filtriert und unter vermindertem Druck bei 35°C eingeengt. Eine Destillation über eine kurze Vigreux-Kolonne [Sdp. 49 - 55°C/0,40 - 0,45 mbar (40 - 45 Pa)] ergab 17,24 g (78%ige Ausbeute) 1-Isobutoxy-2,4,4-trimethyl-3-exomethylen-cyclopenten als farbloses Oel mit einen Gehalt nach GC von 99,4%. ¹H-NMR (250 MHz, d₆-DMSO): u.a. 4,37 (d, J ~ 3 Hz, 2 olefinische H), 3,65 (d, J ~ 7 Hz, 2H, -O-CH₂-CH); IR (Film): 1659,1619 cm⁻¹; Massenspekrum: 194 (M⁺, 65%).

| Mikroanalyse | | |
|---|---|---|
| Ber. | C 80,35% | H 11,41% |
| Gef. | C 80,47% | H 11,32% |

### Beispiel 4

### 1-Isobutoxy-2,4,4-trimethyl-3-exomethylen-cyclohexen (Formel III, worin R³ Wasserstoff, R⁴ Isobutoxy und m 1 bedeuten; zweistufiges Verfahren)

### (i) Herstellung von 1-Isobutoxy-2,4,4-trimethyl-3-hydroxy-3-trimethysilylmethylcyclohexan

In einem mit Magnetrührer, Thermometer, Tropftrichter und Argonbegasung ausgestatteten 750 ml-Vierhalssulfierkolben wurden 450 ml einer 0,8 M-Lösung von Trimethylsilylmethylithium (etwa 360 mmol/1,3 Aeq.) in Pentan [hergestellt aus 7,1 g (1 mol) Lithium-Pulver, 51 g (0,41 mol) Chlormethyltrimethylsilan und 350 ml Pentan analog der in Beispiel 3 (ii)/J. Organomet. Chem. 9, 165 - 168 (1967) beschriebenen Methodik] vorgelegt. Bei etwa -20°C gab man zur Lösung tropfenweise 60 g (0,278 mol) 1-Isobutoxy-2,4,4-trimethyl-cyclohexen-3-on (9,75%ig rein nach GC) in etwa einer Stunde, was eine leicht exotherme Reaktion auslöste. Nach beendeter Zugabe wurde in einem Eisbad bei 0°C eine Stunde weitergerührt, bis gemäss GC kein Edukt mehr vorhanden war.

Nun wurden durch den Tropftrichter 100 ml Wasser langsam zugegeben. Danach wurde die wässrige Phase abgetrennt und zweimal mit je 50 ml, insgesamt 100 ml, Pentan extrahiert. Die vereinigte organische Phase wurde nochmals mit 100 ml gesättigter Natriumchlorid-Lösung gewaschen, mit wasserfreiem Natriumsulfat getrocknet und unter vermindertem Druck eingeengt. Kristallisation aus 125 ml Pentan unter Rühren bei 50°C während etwa einer Stunde ergab nach dem Abnutschen und etwa 18-stündigem Trocknen unter Hochvakuum bei Raumtemperatur 75 g (90%ige Ausbeute) 1-Isobutoxy-2,4,4-trimethyl-3-hydroxy-3-trimethylsilylmethyl-cyclohexen als weisse Kristalle, Smp. etwa 50°C; ¹H-NMR (250 MHz, CDCl₃): u.a. 0,12 (s, 9H, Si(CH₃)₃), 1,80 (t, J = 7 Hz, 2-CH₂), 1,86 (s, 2H, -CH₂-Si), 2,47 (t, J = 7 Hz, 3-CH₂).

Dieses Produkt musste sofort in die nächste Stufe eingesetzt werden, da es sehr instabil ist und sich leicht in die ölige Verbindung der Formel XV umlagert (siehe entsprechende Bemerkung in der allgemeinen Beschreibung).

### (ii) Herstellung von 1-Isobutoxy-2,4,4-trimethyl-3-exomethylen-cyclohexen

In einem mit mechanischem Rührer, Thermometer, Tropftrichter und Argonbegasung ausgestatteten 750 ml-Vierhalssulfierkolben wurden 60 ml einer Kaliumhydrid-Suspension in Oel (etwa 20% Gew./Vol., enthaltend etwa 12 g/0,3 mol KH) vorgelegt. Man wusch das Kaliumhydrid dreimal mit je 25 ml, insgesamt 75 ml, Pentan, wobei nach jedem Waschen das Lösungsmittel dekantiert wurde, und gab 200 ml Tetrahydrofuran zu. Nach Abkühlen des Gemisches auf 5°C mittels eines Eisbades wurde in etwa einer Stunde eine Lösung der obigen 75 g (0,25 mol) 1-Isobutoxy-2,4,4-trimethyl-3-hydroxy-3-trimethylsilylmethyl-cyclohexen in 50 ml Tetrahydrofuran bei einer Temperatur von höchstens 10°C zugetropft. Nun wurde bei 15 - 20°C/eine Stunde und bei Raumtemperatur/eine Stunde nachgerührt (GC-Kontrolle: etwa 96% Produkt und kein Edukt mehr).

Zur Aufarbeitung wurde auf 0°C abgekühlt und 200 ml Wasser vorsichtig zugetropft. Die beiden Phasen wurden nun abgetrennt und die wässrige Phase dreimal mit je 100 ml, insgesamt 300 ml, Pentan extrahiert. Die vereinigten organischen Phasen wurden mit 100 ml gesättigter Natriumchlorid-Lösung gewaschen, mit wasserfreiem Natriumsulfat getrocknet und eingeengt. Der anfallende Rückstand wurde nochmals in 250 ml Pentan aufgenommen, nochmals mit wasserfreiem Natriumsulfat getrocknet, filtriert und unter vermindertem Druck eingeengt. Dies ergab 58 g rohes 1-Isobutoxy-2,4,4-trimethyl-3-exomethylen-cyclohexen als gelbliche Flüssigkeit. Eine Destillation bei 0,1 mbar (10 Pa) über eine kleine 10 cm-Vigreux-Kolonne ergab bei einem Siedepunkt von etwa 55 - 60°C 51,2 g (93%ige Ausbeute) 1-Isobutoxy-2,4,4-trimethyl-3-exomethylen-cyclohexen als farbloses Oel mit einem Gehalt an gewünschtem Produkt von 95% nach GC. ¹H-NMR (C₆D₆, 250 MHz): 0,89 (d, J = 7 Hz, 2 x CH₃), 1,12 (s, 2 x CH₃), 1,42 (t, J = 7 Hz, 2-CH₂), 1,80 [heptett, J = 7 Hz, -CH-(CH₃)₂], 2,03 (t, J - 2 Hz, 5-CH₃), 2,08 (bt, J - 7 Hz, 3-CH₂), 3,28 (d, J = 7 Hz, O-CH₂), 4,97 (d, J = 10 Hz, = CH₂); IR (Film): 1643, 1118 cm⁻¹

| Mikroanalyse: | | |
|---|---|---|
| Ber. | C 80,71% | H 11,61% |
| Gef. | C 80,64% | H 12,01% |

### Beispiel 5

### 1-Isobutoxy-2,4,4-trimethyl-3-exomethylen-cyclohexen (einstufiges Verfahren)

In einem mit mechanischem Rührer, Thermometer, 20 cm-Vigreux-Kolonne mit Destillationsaufsatz und Argonbegasung ausgestatteten 750 ml-Vierhalssulfierkolben wurden 400 ml einer 0,76 M-Lösung von Trimethylsilylmethyllithium (etwa 0,3 mol/1,2 Aeq.) in Pentan [hergestellt aus 8,5 g (1,2 mol) Lithium-Pulver, 50 g (0,4 mol) Chlormethyltrimethylsilan und 300 ml Pentan analog der in Beispiel 3 ((ii)/J. Organomet. Chem. 9,165 - 168 (1967) beschriebenen Methodik] vorgelegt. Bei etwa -20°C gab man zur Lösung innert 30 Minuten tropfenweise 53 g (0,25 mol) 1-Isobutoxy-2,4,4-trimethylcyclohexen-3-on (97,5%ig rein nach GC) in 100 ml Tetrahydrofuran, was eine leicht exotherme Reaktion auslöste. Nun wurde eine Stunde bei 0°C gerührt, anschliessend durch die Vigreux-Kolonne etwa 300 ml Pentan abdestilliert und nach Zugabe von 200 ml Tetrahydrofuran die Destillation weitergeführt, bis ein Siedepunkt von etwa 60°C erreicht wurde (GC-Kontrolle: etwa 90% gewünschtes Produkt, kein Edukt bzw. Zwischenprodukt mehr vorhanden).

Dann wurde auf +5°C abgekühlt, vorsichtig 200 ml Wasser zugegeben und wie bei vorherigem Versuch [Beispiel 4(ii)] aufgearbeitet. Dies ergab 55 g rohes 1-Isobutoxy-2,4,4-trimethyl-3-exomethylen-cyclohexen als bräunliches Oel, welches man unter Hochvakuum bei 0,15 mbar (15 Pa) über eine 10cm-Vigreux-Kolonne destillierte. Bei einem Siedepunkt von 58 - 60°C wurden 39 g (71%ige Ausbeute) 1-Isobutoxy-2,4,4-trimethyl-3-exomethylen-cyclohexen als leicht gelbliche Flüssigkeit erhalten. Gehalt nach GC 94%; spektroskopische Werte wie in Beispiel 4(ii).

### Beispiel 6

### 2,2,4,6,6-Pentamethyl-5,6,7,7a-tetrahydro-5-methyliden-1,3-benzodioxol (Formel III, worin R³ und R⁴ zusammen Isopropylidendioxy und m 1 bedeuten)

### (i) Herstellung von 2,2,4,6,6-Pentamethyl-5,6,7,7a-tetrahydro-5-trimethylsilylmethyl-1,3-benzodioxol-5-ol

In einem mit mechanischem Rührer, Thermometer, Tropftrichter und Argonbegasung ausgestatteten 1,51-Vierhalssulfierkolben wurden etwa 500 ml einer etwa 0,8M-Lösung von Trimethylsilylmethyllithium (etwa 0,4 mol/2.0 Aeq.) in Pentan [hergestellt aus 5,2 g (0,75 mol) Lithium-Pulver, 62,5 g (0,51 mol) Chlormethyltrimethylsilan und 250 ml Pentan analog der in Beispiel 3(ii)/J. Organomet. Chem. 9, 165 - 168 (1967) beschriebenen Methodik] vorgelegt. Bei etwa -20°C gab man zur Lösung innert 30 Minuten tropfenweise 42,0 g (0,2 mol) 2,2,4,6,6-Pentamethyl-5,6,7,7a-tetrahydro-1,3-benzodioxol-5-on in 140 ml Tetrahydrofuran. Danach liess man das Reaktionsgemisch langsam auf 0°C, dann auf Raumtemperatur erwärmen, bei welcher Temperatur 30 Minuten gerührt wurde. Nach beendeter Reaktion wurde erneut auf 0°C abgekühlt und 200 ml Wasser langsam zugetropft. Nach Abtrennung der wässrigen Phase wurde noch zweimal mit 100 ml, insgesamt 200 ml, Hexan extrahiert. Die organischen Phasen wurden vereinigt und mit je 100 ml gesättigter Natriumbicarbonat- und gesättigter Natriumchlorid-Lösung gewaschen, über wasserfreiem Natriumsulfat getrocknet und bei 40°C/30 mbar (3 kPa) eingeengt. Dies ergab 59,6 g rohes 2,2,4,6,6-Pentamethyl-5,6,7,7a-tetrahydro-5-trimethylsilylmethyl-1,3-benzodioxol-5-ol als farblose, feuchte Kristalle. Umkristallisation aus 250 ml Pentan (nach heissem Lösen, Abkühlen auf etwa -20°C) ergab 49,3 g reines Produkt als weisse Kristalle, Smp. 95°C. Aus der Mutterlauge konnten weitere 3,7 g kristallines Produkt, Smp. 95°C, erhalten werden, d.h. es wurden schliesslich 53,0 g (89%ige Ausbeute) 2,2,4,6,6-Pentamethyl-5,6,7,7a-tetrahydro-5-trimethylsilylmethyl-1,3-benzodioxol-5-ol hergestellt. ¹H-NMR (CDCl₃, 250 MHz): u.a. 0,06 (s, 9H, Si(CH₃)₃), 0,98 (s, 6H, C(6)-(CH₃)₂), 1,40 und 1,42 (2s, 6H, C(2)-(CH₃)₂), 4,43 (triplettoid, J ~ 8 Hz, CH-O); Massenspektrum: 281 (M⁻-OH, 5%), 242 (M⁺-Isobutylen, 100%).

| Mikroanalyse: | | |
|---|---|---|
| Ber. | C 64,38% | H 10,13% |
| Gef. | C 64,10% | H 9,95% |

### (ii) Herstellung von 2,2,4,6,6-Pentamethyl-5,6,7,7a-tetrahydro-5-methyliden-1,3-benzodioxol

In einem mit Magnetrührer und Tropftrichter mit aufgesetztem Argonblasenzähler ausgestatteten 500 ml-Rundkolben wurden 23 ml einer 20%igen Suspension von Kaliumhydrid in Oel (enthaltend etwa 3,7g/92 mmol KH) einpipettiert und dreimal mit jeweils 10 ml Hexan gewaschen. Dann gab man 120 ml Tetrahydrofuran dazu und tropfte bei Raumtemperatur eine Lösung von 25,0 g (83 mmol) 2,2,4,6,6-Pentamethyl-5,6,7,7a-tetrahydro-5-trimethylsilylmethyl-1,3-benzodioxol-5-ol in 220 ml Tetrahydrofuran zu und rührte 30 Minuten bei Raumtemperatur (GC-Kontrolle: kein Edukt mehr vorhanden). Dann wurde auf 0°C abgekühlt und 50 ml Wasser langsam zugetropft. Eine analoge Aufarbeitung wie in (i) beschrieben ergab 29,7 g rohes 2,2,4,6,6-Pentamethyl-5,6,7,7a-tetrahydro-5-methyliden-1,3-benzodioxol als gelbliches Oel. Eine Destillation an einer sehr kurzen Vigreux-Kolonne ergab bei einer Siedetemperatur von 50°C/0,02 mbar (2 Pa) 15,6 g (90%ige Ausbeute) reines Produkt als farbloses Oel. ¹H-NMR (CDCl₃, 250 MHz): u.a. 1,12 und 1,23 (2s, je 3H, C(6)-(CH₃)₂), 1,50 und 1,53 (2s, je 3H, C(2)(CH₃)₂), 1,73 (s, 3H, C(4)-CH₃), 4,55 (m, 1H, CH-O-), 4,79 (d, J = 6 Hz, =CH₂); IR (Film): 1693, 1112 cm⁻¹; Massenspektrum: 208 (M⁺, 40), 107(100);

| Mikroanalyse: | | |
|---|---|---|
| Ber. | C 74,96% | H 9,68% |
| Gef. | C 74,70% | H 9,42% |

### Beispiel 7

### 4,6,6-Trimethyl-5,6,7,7a-tetrahydro-5-methyliden-1,3-benzodioxol (Formel III, worin R³ and R⁴ zusammen Methylendioxy und m 1 bedeuten)

### (i,a) Herstellung von 4,6,6-Trimethyl-5,6,7,7a-tetrahydro-1,3-benzodioxol-5-on (unter Verwendung von Formaldehyd-dimethylacetal)

In einem 2l-Rundkolben wurden 85 g (0,5 mol) 2,2,6-Trimethyl-4,5-dihydroxycyclohex-5-en-1-on in 700 ml Ethylacetat und 210 ml (2,4 mol) Formaldehyd-dimethylacetal vorgelegt und 5 g Amberlyst® 15 (H⁺-Form) zugegeben. Dann wurde dem Rundkolben ein Soxhlet (500ml gefüllt mit Molekularsieb 3Å) mit einem Kühler aufgesetzt. Nach insgesamt 10-stündigem Rückfluss wurde das Gemisch vom Katalysator abfiltriert und bei 35°C /62 mbar (6,2 kPa) eingeengt. Nach Destillation über eine 10 cm-Füllkörperkolonne wurden bei Siedepunkt 78 - 84°C /0.3 - 0,15 mbar (30 - 15Pa) 77,5 g (85%ige Ausbeute) eines Oels erhalten, welches beim Abkühlen erstarrte. 5 g dieses Produktes wurden aus 20 ml Pentan bei bis -20°C umkristallisiert. Dies ergab nach Filtration und Trocknung unter Hochvakuum bei Raumtemperatur 4,50 g 4,6,6-Trimethyl-5,6,7,7a-tetrahydro-1,3-benzodioxol-5-on als weisse Kristalle, Smp. 55,5 - 57°C. IR (cm⁻¹): 1690, 1639; Massenspektrum: 182 (M⁺, 30), 126 (100); ¹H-NMR (CDCl₃, 400 MHz): 1,16, 1,21 (2s, je 3H), 1,73 (s, 3H), 1,91 (t, J = 19 Hz, 1H), 2,27 (q, J₁ = 19 Hz, J₂ = 9 Hz, 1H), 4,6 (m, 1H), 5,32 (s, 1H), 5,61 (s, 1H),

| Mikroanalyse: | | |
|---|---|---|
| Ber. | C 65,92% | H 7,74% |
| Gef. | C 65,83% | H 7,79% |

### (i,b) Herstellung von 4,6,6-Trimethyl-5,6,7,7a-tetrahydro-1,3-benzodioxol-5-on (unter Verwendung von Paraformaldehyd)

In einem 250 ml Rundkolben wurden 10,0 g (58 mmol) 2,2,6-Trimethyl-4,5-dihydroxy-cyclohex-5-en-1-on in 100 ml Ethylacetat vorgelegt und 3,5 g (116 mmol) Paraformaldehyd und 500 mg Amberlyst® 15 (H⁺-Form) zugegeben. Ueber eine 30 cm Vigreux-Kolonne wurde nun kontinuierlich das entstehende Wasser azeotrop mit Ethylacetat abdestilliert (Rücklaufverhältnis 10:1). Nach 2½ Stunden wurden 50 ml Essigester in den Kolben zugegeben. Nach einer weiteren Stunde war die Reaktion nach Kontrolle durch Dünnschichtchromatographie beendet. Zur Aufarbeitung wurde die Reaktionslösung filtiert und unter vermindertem Druck eingeengt. Eine Kurzwegdestillation unter Hochvakuum bei 0,3 mbar (30 Pa) und 100°C Badtemperatur ergab 9,8 g (93%ige Ausbeute) öliges Destillat, das sich beim Abkühlen verfestigte. Das Produkt war identisch mit dem unter (i,a) beschriebenen Produkt.

### (ii) Herstellung von 4,6,6-Trimethyl-5,6,7,7α-tetrahydro-5-trimethylsilylmethyl-1,3-benzodioxol-5-ol

24,7 ml (20,6 g, 0,17 mol) Chlormethyltrimethylsilan wurden in 150 ml Pentan mit 2,92 g (0,42 mol) Lithiumpulver umgesetzt (gemäss der in Beispiel 3 beschriebenen Methodik). Die resultierende Lösung von Trimethylsilylmethyllithium (etwa 0,14 mol) in Pentan wurde in einem 500 ml Kolben unter Argonbegasung vorgelegt. Bei -20°C gab man dazu eine Lösung von 20 g (0,11 mol) 4,6,6-Trimethyl-5,6,7,7a-tetrahydro-1,3-benzodioxol-5-on in 25 ml Tetrahydrofuran innert etwa 20 Minuten. Nach weiteren 20 Minuten wurde langsam auf Raumtemperatur erwärmt, dann erneut auf 0°C abgekühlt. Man tropfte danach 90 ml Wasser zu. Anschliessend wurde das Wasser in einem Scheidetrichter abgetrennt und zweimal mit je 100 ml Pentan extrahiert. Nach dem Waschen der Pentanphasen der Reihe nach mit Natriumbicarbonat- und Natriumchlorid-Lösung wurde über wasserfreiem Natriumsulfat getrocknet und eingeengt. Das Rohprodukt (31,9 g) wurde nun zur Kristallisation in 60 ml Pentan gelöst und auf -25°C abgekühlt. Die resultierenden weissen Kristalle wurden abfiltriert, mit wenig Pentan bei -20°C gewaschen und unter Hochvakuum bei Raumtemperatur getrocknet. Dies ergab 30,5 g (fast 100%ige Ausbeute) 4,6,6-Trimethyl-5,6,7,7a-tetrahydro-5-trimethylsilylmethyl-1,3-benzodioxol-5-ol als weisse Kristalle, Smp. 63 - 64,5°C. IR (Nujol, cm⁻¹): 3510 (OH); Massenspektrum: 253 (M⁺-OH, 5), 214 (M-C₄H₈); ¹H-NMR (C₆D₆, 400 MHz): u.a. 0,27 (s, 9H), 4,9 (s, 1H), 5,19 (s, 1H).

| Mikroanalyse: | | |
|---|---|---|
| Ber. | C 62,18% | H 9,69% |
| Gef. | C 62,07% | H 9,51% |

### (iii) Herstellung von 4,6,6-Trimethyl-5,6,7,7a-tetrahydro-5-methyliden-1,3-benzodioxol

In einem mit Magnetrührer, Tropftrichter und Argonbegasung ausgestatteten 750 ml-Vierhalssulfierkolben wurden 23 ml (etwa 1 Aeq.) Kaliumhydrid (20% in Oel) vorgelegt und dreimal mit Hexan gewaschen, und 120 ml Tetrahydrofuran wurden zugegeben. Bei 0°C wurden dazu eine Lösung vom 30,4 g (0,11 mol) 4,6,6-Trimethyl-5,6,7,7a-tetrahydro-5-trimethylsilylmethyl-1,3-benzodioxol-5-ol in 200 ml Tetrahydrofuran zugetropft. Anschliessend liess man die Suspension 2 Stunden bei 20 - 30°C rühren. Dann wurde wieder auf 0°C abgekühlt, und es wurden vorsichtig 200 ml Wasser zugetropft. Anschliessende dreimalige Extraktion mit je 100 ml Hexan, Waschen mit gesättigter Natriumchloridlösung und Trocknung über wasserfreiem Natriumsulfat ergab nach Entfernung des Lösungsmittels unter vermindertem Druck 18,7 g gelbes Oel, welches unter Hochvakuum kurzwegdestilliert wurde. Bei 77 - 85°C/0,75 mbar (7,5 kPa) wurden 15,6 g (74%ige Ausbeute) 4,6,6-Trimethyl-5,6,7,7a-tetrahydro-5-methyliden-1,3-benzodioxol als farbloses Oel erhalten. Der Gehalt am gewünschten Produkt nach GC (Flächen-%) betrug 96%. IR (Film, cm⁻¹): 1697, 1600; Massenspektrum: 180 (M⁺, 50), 107 (100); 1H-NMR (C₆D₆, 400 MHz): u.a. 0,83 und 0,95 (2s, je 3H), 1,78 (bs, 3H), 3,9 (m, 1H), 4,59 (s, 1H), 4,70 (s, 1H), 4,75 (s, 1H), 4,91 (s 1H).

| Mikroanalyse: | | |
|---|---|---|
| Ber. | C 73,30% | H 8,95% |
| Gef. | C 73,08% | H 9,21% |

### Beispiel 8

### 4,6,6-Trimethyl-5,6,7,7a-tetrahydro-5-methyliden-1,3-benzodioxol ("Durchprozess")

Die aus 8,7 g (1,25 mol) Lithiumpulver und 74 ml (61,6 g, 0,5 mol) Chlormethyltrimethylsilan in 450 ml Pentan hergestellte Lösung von Trimethylsilylmethyllithium (etwa 0,43 mol) wurde in einem mit Magnetrührer, Tropftrichter, Kühler und Argonbegasung ausgestatteten 750ml-Vierhalssulfierkolben vorgelegt. Dazu tropfte man nun bei -20°C eine Lösung von 60,0 g (0,33 mol) 4,6,6-Trimethyl-5,6,7,7a-tetrahydro-1,3-benzodioxol-5-on in 75 ml Tetrahydrofuran innert 30 Minuten zu. Dann wurde langsam auf Raumtemperatur erwärmt und anschliessend das Pentan bis zum Siedepunkt 62°C über eine Vigreux-Kolonne abdestilliert. Das abdestillierende Pentan wurde mit 500 ml Tetrahydrofuran kontinuierlich ersetzt. Am Schluss wurde 12 Stunden am Rückfluss erhitzt, und zwar unter GC-Kontrolle. Anschliessend wurde auf 0°C abgekühlt, 200 ml Wasser zugetropft, die Phasen abgetrennt und die Wasserphase dreimal mit je 100 ml Pentan extrahiert. Die gesamte organische Phase wurde mit je 150 ml gesättigter Natriumbicarbonat- und Natriumchlorid-Lösung gewaschen, über wasserfreiem Natriumsulfat getrocknet, filtriert und eingeengt. Dies ergab 67,5 g gelbes Oel, welches man über eine 10 cm-Füllkörperkolonne destillierte. Bei einer Siedetemperatur von 38°C/0,04 mbar (4 Pa) wurden 49,0 g (82%ige Ausbeute) 4,6,6-Trimethyl-5,6,7,7a-tetrahydro-5-methyliden-1,3-benzodioxol als farbloses Oel erhalten. Die analytischen Daten des Produktes waren gleich wie diejenigen des Produktes des Beispiels 7.

### C. Herstellung der symmetrischen, endständig ringsubstituierten Polyene (Verbindungen der Formel I)

### Beispiel 9

### 2,2'-Dinor-canthaxanthin (Formel I, worin R¹ Wasserstoff, m 0 und n 1 bedeuten; "Durchprozess" II + III → [IV] → I)

In einem mit Magnetrührer ausgestatteten 100 ml-Rundkolben wurden 3,4 g (8,6 mmol) Crocetindialdehyd-dimethylacetal und 5,1 g (25 mmol) 1-Isobutoxy-2,4,4-trimethyl-3-exomethylen-cyclopenten (98%ig rein nach GC) in 60 ml Acetonitril vorgelegt und 400 mg (1,8 mmol, 20 mol%) wasserfreies Zinkbromid zugegeben, und das Reaktionsgemisch wurde während 16 Stunden bei 50°C gerührt. Nach dieser Reaktionsdauer ergab eine Analyse nach Dünnschichtchromatographie mit einem 9:1-Gemisch von Toluen und Ethylacetat als Laufmittel, dass sowohl noch Crocetindialdehyddimethylacetal (R_{f} = etwa 0,6) als auch das Reaktionsprodukt der Formel IV (R_{f} = etwa 0,3) anwesend waren.

Zur erhaltenen dunklen Lösung wurden dann 30 ml Ethylacetat zugegeben, und das Reaktionsgemisch wurde auf 0°C abgekühlt und zur eventuellen Hydrolyse mit 5 ml eines 9 : 1-Gemisches von Essigsäure und Wasser versetzt. Es wurde dann eine Stunde bei dieser Temperatur weitergerührt.

Zur Aufarbeitung wurde das Gemisch mit etwa 300 ml Ethylacetat verdünnt und das Ganze nacheinander mit je etwa 100 ml Wasser, gesättigter Natriumbicarbonatlösung und gesättigter Natriumchloridlösung gewaschen. Man trocknete die organische Phase mit wasserfreiem Natriumsulfat und engte sie unter vermindertem Druck bei 35°C ein. Dies ergab 6,8 g rohes 8,8'-Dimethoxy-7,8,7',8'-tetrahydro-2,2'-dinor-canthaxanthin (diesbezügliche Verbindung der Formel IV, worin u.a. R² Methyl bedeutet) als dickflüssiges, rotes Oel.

Man löste dieses Oel in 50 ml Ethanol und versetzte die Lösung mit 5,7 ml einer 1,6-molaren Lösung von Natriumethylat in Ethanol (enthaltend 9 mmol NaOC₂H₅). Das Reaktionsgemisch wurde 2 Stunden bei 80°C gerührt und dann auf 0°C abgekühlt. Die angefallenen dunklen Kristalle wurden abgenutscht und unter Hochvakuum bei Raumtemperatur getrocknet, was 3,7 g rohes 2,2'-Dinor-canthaxanthin als dunkelviolette Kristalle ergab. Man nahm die Kristalle in 75 ml Isopropanol auf und erhitzte das Gemisch 16 Stunden bei Rückflusstemperatur. Anschliessend wurde aus Methylenchlorid/Isopropanol (1:1) umkristallisiert, wobei man den grössten Teil des Methylenchlorids unter vermindertem Druck absog. Nach dem Filtrieren, Waschen und Trocknen unter Hochvakuum bei Raumtemperatur erhielt man 2,5 g (54%ige Ausbeute bezogen auf das Diacetal der Formel II) 2,2'-Dinor-canthaxanthin als dunkelviolette Kristalle, Smp. 223 - 224°C; UV (Cyclohexan/2% Methylenchlorid): 527 nm (logE = 5,02), 494 nm (logE = 5,13), 468 nm (logE = 5,02), 319 nm (logE = 4,45); ¹H-NMR (400 MHz, CDCl₃): u.a. 1,33 (s, 2 x CH₃), 1,90, 2,00, 2,03 (3s, 3 x CH₃), 2,35 (s, 3-CH₂); IR (KBr): 1685 cm⁻¹.
Massenspektrum: 536 (M⁺, 100%).

### Beispiel 10

### Herstellung und Isolierung des Zwischenproduktes 8,8'-Dimethoxy-7,8,7',8'-tetrahydro-2,2'-dinor-canthaxanthin (Formel IV, worin R¹ Wasserstoff, R² Methyl, m 0 und n 1 bedeuten)

In einem mit Magnetrührer ausgestatteten 50 ml Rundkolben wurde ein Gemisch von 1,18 g (3 mmol) Crocetindialdehyd-dimethylacetal, 1,76 g (9 mmol) 1-Isobutoxy-2,4,4-trimethyl-3-exomethylen-cyclopenten und 135 mg (0,6 mmol, 20 mol%) wasserfreiem Zinkbromid in 25 ml Acetonitril 18 Stunden bei 40°C und anschliessend 6 Stunden bei 50°C gerührt, währenddessen die gleiche Kontrolle durch Dünnschichtchromatographie wie in Beispiel 9 beschrieben vorgenommen wurde. Dann wurde auf 0°C abgekühlt und 2 ml eines 9 : 1-Gemisches von Essigsäure und Wasser, gefolgt von 10 ml Ethylacetat zugegeben. Nach zweistündigem Rühren des Gemisches bei 0°C wurde eine übliche (wie in Beispiel 9 beschrieben) Aufarbeitung, gefolgt von einer Säulenchromatographie an 150 g Kieselgel (0,04 - 0,063 mm) mit 9 : 1-Toluen/Ethylacetat sowie einer Digerierung aus Ethanol bei 50°C vorgenommen. Dies ergab 230 mg (etwa 13%ige Ausbeute) 8,8'-Dimethoxy-7,8,7',8'-tetrahydro-2,2'-dinor-canthaxanthin als rote Kristalle, Smp. 163 - 164°C.; ¹H-NMR (CDCl₃, 400 MHz): u.a. 1,18, 1,21 (2s, 12H, C(1)-(CH₃)₂, C(1')-(CH₃)₂), 2,29 (s, 4H, C(3)H₂, C(3')H₂), 2,45 (d x d, J₁ = 14 Hz, J₂ = 5 Hz, 2H, C(7)H, C(7')H), 2,69 (d x d, J₁ = 14 Hz, J₂ = 7 Hz, 2H, C(7)H, C(7')H), 3,13 (s, 6H, 2 x OCH₃), 3,75 (d x d, J₁ = 7 Hz, J₂ = 5 Hz, C(8)H, C(8')H), 6,05 - 6,7 (m, etwa 10, olefinische H);
Massenspektrum: 600,5 (M⁺, 25%).

### Beispiel 11

### Canthaxanthin (Formel I, worin R¹ Wasserstoff und m und n beide 1 bedeuten)

### (i) Herstellung von 3,4,3',4'-Tetradehydro-7,8,7',8'-tetrahydro-4,4'-diisobutoxy-8,8'-dimethoxy-β,β'- carotin (Formel V, worin R² Methyl, R⁴ Isobutoxy und m und n beide 1 bedeuten)

In einem mit Magnetrührer, Thermometer und Calciumchlorid-Rohr versehenen 50 ml-Zweihalskolben wurden 1,7 g (4,3 mmol) Crocetinaldehyd-dimethylacetal in 20 ml Acetonitril und 5 ml Ethylacetat suspendiert. Bei 0 - 5°C wurden 2,7 g (12,3 mmol) 1-Isobutoxy-2,4,4-trimethyl-3-exomethylen-cyclohexen und 120 mg (20 mol%) wasserfreies Zinkchlorid zugegeben. Das Eisbad wurde nach kurzer Zeit entfernt und die orange Suspension etwa 20 Stunden bei Raumtemperatur gerührt. Zur daraus entstandenen zitronengelben Suspension wurde 0,5 ml Triethylamin zugegeben, und das Gemisch wurde auf 0°C abgekühlt und nach einer Stunde abgenutscht. Nach Trocknung unter Hochvakuum bei Raumtemperatur wurden 2,6 g (etwa 82%ige Ausbeute) der im Titel genannten Verbindung als hellgelbes Pulver, Smp. 155 - 166°C, erhalten.

Für die analytischen Daten wurde dieses Produkt zweimal je 30 Minuten in 25 ml Aceton rückflussiert, dann 2 Stunden auf-10°C gekühlt und abgenutscht. Nach Trocknung unter Hochvakuum bei Raumtemperatur wurden 1,4 g 3,4,3',4'-Tetradehydro-7,8,7',8'-tetrahydro-4,4'-diisobutoxy-8,8'-dimethoxy-β,β'-carotin als hellgelbes Pulver, Smp. 166 - 173°C, erhalten. UV (Cyclohexan/2% Chloroform): 430 nm (logE = 5,16), 404 nm (logE = 5,15), 383 nm (logE = 4,93), 365 nm (logE = 4,60; ¹H-NMR (C₆D₆, 400 MHz): u.a. 0,89 (d, J = 7 Hz, 12H, 2 x CH(CH₃)₂), 1,20 (s, 6H, C(1)-CH₃, C(1')-CH₃), 1,23 (s, 6H, C(1')-CH₃, C(1')-CH₃), 3,09 (s, 6H, 2 x OCH₃), 3,33 (d, J = 7 Hz, 4H, 2 x O-CH₂-), 4,59 (t, J ~ 5 Hz, 2H, 2 x C(3)H); IR (KBr): kein C = O, 1650, 1089 cm⁻¹ (C-O-C); Massenspektrum: 740,8 (M⁺, ≤ 1%), 533,4 (15%), 326,1 (100%).

### (ii) Herstellung von 7,8,7',8'-Tetrahydro-8,8'-dimethoxy-canthaxanthin (Formel IV, worin R¹ Wasserstoff, R² Methyl, und m und n beide 1 bedeuten)

In einem mit Magnetrührer, Thermometer und Argonbegasung ausgestatteten 50 ml-Zweihalskolben wurde 1,00 g (1,35 mmol) 3,4,3',4'-Tetradehydro-7,8,7',8'-tetrahydro-4,4'-diisobutoxy-8,8'-dimethoxy-β,β'-carotin (Smp. 166 - 173°C) in 10 ml Methanol suspendiert und mit 1 ml 50%iger wässriger Essigsäure, gefolgt von 30 mg p-Toluensulfonsäure-Monohydrat, versetzt. Das Gemisch wurde 3 Stunden bei 35 - 40°C gerührt. Nun gab man 2 ml Wasser zu, kühlte auf 0°C ab und filtrierte ab. Nach Trocknung unter vermindertem Druck wurden 800 mg (etwa 94%ige Ausbeute) der im Titel genannten Verbindung als gelb-oranges Pulver, Smp. 183 - 189°C, erhalten.

Für die analytischen Daten wurden 750 mg des erhaltenen Produktes an Silikagel (0,04 - 0,063 mm) mit einem 9 : 1-Gemisch von Methylenchlorid und Diethylether gereinigt. Die reinen Fraktionen wurden eingeengt und der Rückstand 2 Stunden in 10 ml Methanol bei Rückflusstemperatur digeriert. Nach Abkühlen, Filtrieren und Trocknen unter Hochvakuum bei Raumtemperatur wurden 370 mg reines 7,8,7',8'-Tetrahydro-8,8'-dimethoxy-canthaxanthin als hellgelbes Pulver, Smp. 200 - 203°C, erhalten; Gehalt nach HPLC: 96,5 % (Flächen-%); UV (Cyclohexan / 3% Chloroform): 429 nm (logE = 5,14), 403 nm (logE = 5,13), 382 nm (logE = 4,90), 365 nm (logE = 4,59); ¹H-NMR (CDCl₃), 400 MHz): u.a. 1,14 (s = 6H, C(1)-CH₃, C(1')-CH₃), 1,19 (s, 6H, C(1)-CH₃, C(1')-CH₃), 2,40 (d x d, J₁ = 14 Hz, J₂ = 4 Hz, 2H, C(7)-H, C(7')-H), 2,65 (d x d, J₁ = 14 Hz, J₂ = 7 Hz, 2H C(7)H, C(7')H), 2,50 (t, J ~ 7 Hz, 4H, C(3)H₂, C(3')H₂), 3,11(s, 6H, 2 x OCH₃), 3,70 (d x d, J₁ = 14 Hz, J₂ = 7 Hz, 2H, 2 x CH-OCH₃), 6,0 - 6,7 (m, 10 olefinische H); IR (KBr): 1660 cm⁻¹ (C=O); Massenspektrum: 628,5 (M⁺, 20), 477,3 (100).

| Mikroanalyse: | | | |
|---|---|---|---|
| Ber. | C 79,62% | H 9,63% | (mit 0,26% H₂O) |
| Gef. | C 79,43% | H 9,63% | (H₂O, 0,26%) |

### Beispiel 12

### Canthaxanthin (Formel I, worin R¹ Wasserstoff und m und n beide 1 bedeuten; "Durchprozess")

### (i) Herstellung von 3,4,3',4'-Tetradehydro-7,8,7',8'-tetrahydro-4,4'-diisobutoxy-8,8'-dimethoxy-β,β'-carotin (Formel V, worin R² Methyl, R⁴ Isobutoxy und m und n beide 1 bedeuten)

In einem mit Magnetrührer, Thermometer und Argonbegasung versehenen 100 ml-Zweihalsrundkolben wurden 60 mg (0,4 mmol, 5 Mol%) wasserfreies Eisen(III)chlorid und 5 Tropfen 1-Isobutoxy-2,4,4-trimethyl-3-exomethylen-1-cyclohexen in einer Mischung von 25 ml Methylenchlorid und 25 ml Acetonitril vorgelegt und das Gemisch bei Raumtemperatur 2 Stunden gerührt. Dann wurde auf -20°C abgekühlt und nacheinander 3,0 g (7,5 mmol) Crocetindialdehyd-dimethylacetal (HPLC:≥97%ig rein) und 4,0 g (19 mmol, 2,6 Aeq) 1-Isobutoxy-2,4,4-trirnethyl-3-exoinethylen-1-cyclohexen zugegeben. Man rührte das Gemisch 2 Stunden bei -20°C bis -15°C und weitere 2 Stunden bei -10°C bis -5°C. Es wurde nochmals 1,0 g (4,6 mmol) 1-Isobutoxy-2,4,4-trimethyl-3-exomethylen-1-cyclohexen zugegeben und nochmals 2 Stunden bei -10° bis -5°C gerührt. Dann wurde mit 0,5 ml Triethylamin neutralisiert, 20 ml Methanol zugegeben und das Methylenchlorid unter vermindertem Druck (350 mbar / 35 kPa) bei 20° - 30°C entfernt. Die resultierenden orangen Kristalle wurden nun 2 Stunden bei -5°C gekühlt. Nach dem Abnutschen, Waschen (Methanol, 0°C) und 18-stündigem Trocknen unter Hochvakuum bei Raumtemperatur wurden 5,3 g (etwa 95%ige Ausbeute) 3,4,3',4'-Tetradehydro-7,8,7',8'-tetrahydro-4,4'-diisobutoxy-8,8'-dimethoxy-β,β-carotin als orange Kristalle erhalten und sofort in die nächste Reaktion (ii) eingesetzt.

### (ii) Herstellung von 7,8,7',8'-Tetrahydro-8,8'-dimethoxy-canthaxanthin (Formel IV, worin R¹ Wasserstoff, R² Methyl, und m und n beide 1 bedeuten)

In einem mit Magnetrührer, Thermometer und Argonbegasung ausgestatteten 100 ml-Zweihalsrundkolben wurden 5,2 g (etwa 7 mmol) 3,4,3',4'-Tetradehydro-7,8,7',8'-tetrahydro-4,4'-diisobutoxy-8,8'-dimethoxy-β,β'-carotin in 35ml Methanol suspendiert. Zur Suspension gab man 5 ml 50%ige wässrige Essigsäure und eine kleine Spatelspitze p-Toluensulfonsäure-Monohydrat, und man rührte das Gemisch 3½ Stunden bei 40 - 45°C. Nach Abkühlen des Gemisches auf 3°C gab man 15 ml Wasser zu, und nach 30 Minuten wurde unter Verwendung einer P3-Fritte abgenutscht und mit 10 ml Methanol bei -20°C gewaschen. Nach Trocknen des Produktes unter Hochvakuum bei Raumtemperatur erhielt man 4,0 g der im Titel genannten Verbindung als orange Kristalle, Smp. 169 - 179°C. Durch Lösen der Kristalle in 30 ml Methylenchlorid und Austausch mit 20 ml Methanol unter vermindertem Druck [wie unter (i) beschrieben] wurde umkristallisiert. Dies ergab 3,0 g (68%ige Ausbeute) 7,8,7',8'-Tetrahydro-8,8'-dimethoxy-canthaxanthin als orange Kristalle, Smp. 188 - 201°C [Isomerengemisch; spektroskopische Daten: vgl. Beispiel 11 (ii)]. Diese wurden sofort in die nächste Reaktion (iii) eingesetzt.

### (iii) Herstellung von Canthaxanthin

In einem mit Magnetrührer, Thermometer und Argonbegasung versehenen 50ml-Zweihalsrundkolben wurden 2,8 g (4,5 mmol) 7,8,7,8'-Tetrahydro-8,8'-dimethoxycanthaxanthin in 30 ml Methylenchlorid gelöst. Dann wurde auf -15°C abgekühlt und unter Rühren 1 ml (etwa 9 mmol) 48%ige wässrige Bromwasserstoffsäure zugegeben. Nach Beendigung der Abspaltung (nach etwa 1¼ Stunden; HPLC-Kontrolle) wurden 10 ml (etwa 10 mmol) 1N-Natriumhydroxidlösung auf einmal zugegeben, und das Gemisch wurde 15 Minuten bei 0°C gerührt. Dann wurde die Wasserphase abgetrennt und zweimal mit je 10ml Methylenchlorid extrahiert. Man wusch die vereinigte organische Phase mit 15 ml gesättigter Natriumbicarbonatlösung, trocknete sie über wasserfreiem Natriumsulfat und engte sie auf etwa 20 ml ein. Nun wurde über ein Polster von 20 g Kieselgel filtriert und mit Methylenchlorid/Diethylether (9:1) nachgewaschen. Das filtrierte Produkt wurde nun unter vermindertem Druck bei etwa 100 mbar (10 kPa) eingeengt und gleichzeitig gegen 20 ml Heptan ausgetauscht.

Das E/Z-Verhältnis vor der Isomerisierung war: (all E): 77%, (9Z+13Z): 13%.

Zur Isomerisierung wurde nun am Rückfluss bei etwa 100°C 7 Stunden gekocht, dann auf Raumtemperatur abgekühlt und abfiltirert. Nach 4-stündiger Trocknung bei 70°C unter Hochvakuum wurden 2,3 g (etwa 82%ige Ausbeute) rohes Canthaxanthin als braun-violett Kristalle mit einem HPLC-Gehalt (Flächen-%) von 90,5% (all E) erhalten. Zur Umkristallisation wurde das Rohprodukt in 30 ml Methylenchlorid gelöst und bei etwa 100 mbar (10 kPa) gegen 15 ml Aceton ausgetauscht. Nach 2-stündiger Abkühlung bei -25°C wurde filtriert, mit Aceton bei 0°C gewaschen und 4 Stunden unter Hochvakuum bei 70°C getrocknet. Dies ergab 2,0 g (76%ige Ausbeute) Canthaxanthin als tiefviolette Kristalle, Smp. 207 - 208°C; HPLC-Gehalt (Flächen-%):

| | |
|---|---|
| (all E) - Canthaxanthin: | 95,7% |
| (9Z+13Z)-Canthaxanthin: | 1,6% |
| 8'-Apocanthaacanthinal: | 2,1% |

UV (Cyclohexan/3% CHCl₃): 470 nm (logE = 5,09); IR (KBr):1657 cm¹; Massenspektrum: 564 (M⁺, 22); ¹H-NMR (400 MHz, CDCl₃): 1,20 (s, 12H), 1,85 (t. J ~ 8 Hz, 4H), 1,87 (s, 6H), 1,99 und 2,00 (2s, 12H), 2,50 (t, J ~ 7 Hz, 4H), 6,2 - 6,7 (div. m, 14H).

### Beispiel 13

### Herstellung und Isolierung des Zwischenproduktes 8,8'-Dimethoxy-7,8,7',8'-tetrahydro-3,3'-dihydroxy-4,4'-diketo-β-β'- carotin (Formel IV, worin R¹ Hydroxy, R² Methyl und m und n beide 1 bedeuten)

In einem mit Magnetrührer und Argonbegasung ausgestatteten 25 ml-Rundkolben wurden 582 mg (1,5 mmol) Crocetindialdehyd-dimethylacetal (HPLC: > 97%ig rein) und 937 mg (4,5 mmol) 2,2,4,6,6- Pentamethyl-5,6,7,7a-tetrahydro-5-methyliden-1,3-benzodioxol in 8 ml Acetonitril vorgelegt. Nach Zugabe von 25 mg (0,2 mmol) wasserfreiem Zinkchlorid wurde die Reaktionslösung 16 Stunden bei Raumtemperatur gerührt, und zwar unter Kontrolle durch Dünnschichtchromatographie. Die resultierende dunkelrote Lösung wurde nun eingeengt und das erhaltene Oel an 50 g Kieselgel (0,04 - 0,063 mm) mit Methylenchlorid /Diethylether (9:1) als Lösungsmittel gereinigt. Dies lieferte 331 mg (33%ige Ausbeute) orange Kristalle. Zur weiteren Reinigung für die spektroskopischen Daten wurden sie zu heissem Methanol digeriert, auf -20°C abgekühlt, filtriert und unter Hochvakuum getrocknet. Dies ergab 190 mg 8,8'-Dimethoxy-7,8,7',8'-tetrahydro-3,3'-dihydroxy-4,4'-diketo-β,β'-carotin als hellorange Kristalle, Smp. 171°C; HPLC: 99,5%ig rein; UV (Cyclohexan/3% CHCl₃): 429 nm (logE = 5,12), 403 nm (logE = 5,11), 382 nm (logE = 4,90); IR (KBr): 1657 cm⁻¹; ¹H-NMR (CDCl₃, 400 MHz): u.a. 1,17, 1,23, 1,24, 1,28 (4s, 12H), 3,09, 3,10 (2s, 2 x OCH₃), etwa 3,7 (m, 4H), 4,3 (m, 2H), 6,1 - 6,7 (10 olefinische H).

| Mikroanalyse: | | |
|---|---|---|
| Ber. | C 76,33% | H 9,15% |
| Gef. | C 76,13% | H 9,18% |

### Beispiel 14

### Herstellung von 3,3'-Dihydroxy-4,4'-diketo-β-β'- carotin, d.h. von Astaxanthin (Formel I, worin R¹ Hydroxy und m und n beide 1 bedeuten; "Durchprozess" II + III → I)

In einem mit Magnetrührer und Argonbegasung ausgestatteten 25 ml-Rundkolben wurden 580 mg (1,5 mmol) Crocetindialdehyd-dimethylacetal (HPLC: > 97%ig rein) und 940 mg (4,5 mmol) 2,2,4,6,6- Pentamethyl-5,6,7,7a-tetrahydro-5-methyliden-1,3-benzodioxol in 8 ml Acetonitril vorgelegt. Nach Zugabe von 50 mg (0,4 mmol) wasserfreiem Zinkchlorid wurde die Reaktionslösung 2½ Stunden bei Raumtemperatur gerührt, und zwar unter Kontrolle durch Dünnschichtchromatographie. Die dunkel gefärbte Suspension wurde auf -20°C abgekühlt, und 5 Tropfen 37%ig wässrige Salzsäure wurden zugegeben. Nach 5 Minuten wurde auf 0°C erwärmt und 15 Minuten bei dieser Temperatur weitergerührt. Anschliessend wurde die Reaktionslösung auf 100ml Wasser gegossen und dreimal mit je 50 ml Methylenchlorid extrahiert. Das so erhaltene dunkelrote, ölige Rohprodukt wurde an 70 g Kieselgel (0,04 - 0,063 mm) mit Methylenchlorid /Diethylether (5:1) chromatographiert. Man erhielt auf diese Weise 358 mg (40%ige Ausbeute) Astaxanthin als dunkelrote Kristalle (nach Dünnschichtchromatographie einheitlich). Für die spektroskopischen Daten wurden 320 mg in Methanol heiss digeriert, abgekühlt, filtriert und unter Hochvakuum getrocknet. Dies ergab 198 mg Astaxanthin als glänzende, tiefviolette Kristalle, Smp. 212-218°C. IR (KBr): 1657,1610 cm⁻¹; Massenspektrum: 596,5 (M⁺, 40); ¹H-NMR (CDCl_{3,} 400 MHz): 1,29,1,32 (2s, 2 x 6H), 1,84 (t, J = 12 Hz, 2H), 1,94 (s, 2 x 3H), 2,00, 2,03 (2s, 2 x 6H), 2,16 (q, J = 6Hz, 2H), 3,68 (d, J - 1-2 Hz, 2H), 4,8 (m, 2H), 6,2-6,7 (14 olefinische H).

### Beipiel 15

### Herstellung von 8,8'-Diethoxy-7,8,7',8'-tetrahydro-3,3'-dihydroxy-4,4'-diketo-β,β'-carotin (Formel IV, worin R¹ Hydroxy, R² Ethyl und m und n beide 1 bedeuten)

In einem mit Magnetrühren und Argonbegasung ausgestatteten 50 ml-Rundkolben wurden 20 ml Methylenchlorid vorgelegt und etwa 40 mg (4 Tropfen; 0,4 mmol) Acetondimethylacetal und etwa 25 mg (2 Tropfen; 0,2 mmol, 8 Mol%) Bortrifluorid-diethyletherat zugegen. Nachdem diese Lösung eine Stunde bei Raumtemperatur gerührt worden war (zur Entfernung des Restwassers vom Lösungsmittel), kühlte man auf -25°C ab, gab nacheinander 1,11 g (2,50 mmol) Crocetindialdehyd-diethylacetal und 1,30 g (7,2 mmol) 4,6,6-Trimethyl-5,6,7,7a-tetrahydro-5-methyliden-1,3-benzodioxol ohne Lösungsmittel zu und liess 7 Stunden bei -20 bis -25°C rühren, und zwar unter Kontrolle durch HPLC.

Zur Hydrolyse gab man bei -20°C 4 ml 90%ige wässrige Essigsäure zu, und man rührte das Gemisch 10 Minuten bei Raumtemperatur. Dann wurde auf Wasser gegossen und zweimal mit je 100 ml Hexan extrahiert, mit gesättigter Natriumbicarbonat- und gesättigter Natriumchloridlösung gewaschen, mit wasserfreiem Natriumsulfat getrocknet, filtiert und eingeengt. Dies ergab 2,80 g rotes Oel, welches man an 100 g Kieselgel (0,04 - 0,63 mm) mit Methylenchlorid/Diethylether (9:1) chromatographierte. Dabei konnte 1,00 g (58%ige Ausbeute) 8,8'-Diethoxy-7,8,7',8'-tetrahydro-3,3'-dihydroxy-4,4'-diketo-β,β'-carotin als rötlicher Feststoff isoliert werden; HPLC-Gehalt: 98,1% (Flächen-%). Für die spektroskopischen Daten wurde diese Substanz in 15 ml Methanol heiss digeriert, auf -20°C abgekühlt, filtiert und unter Hochvakuum getrocknet. Dabei wurden 620 mg orange Kristalle, Smp. 140 - 150°C, mit einem Gehalt nach HPLC von 98,8% (Flächen-%) erhalten.

UV (Cyclohexan/3% CHCl₃):429 nm (logE = 5,14), 403 nm (logE = 5,13), 382 nm (logE = 4,91); IR (Nujol): 1664 cm⁻¹, 1607 cm⁻¹ Massenspektrum: 688,6 (M⁺, 5), 521,4 (30); ¹H-NMR (CDCl₃, 400 MHz; Diastereomeren-Gemisch): u.a. 2,3 (d x d, J₁ = 14 Hz, J₂ = 3 Hz, 1H), 2,45 (d x d, J₁= 14 Hz, J₂ = 4,5 Hz, 1H), 2,6 (d x d, J₁ = 14 Hz, J₂ = 7,5 Hz, 1H), 2,7 (d x d, J₁,= 14 Hz, J₂ = 8,5 Hz, 1H), 3,1 und 3,35 (2m, 4H, 2 x OCH₂), 3,65 (2d, J ~ 6 Hz, 2 x OH), 3,8 (m, 2H, CH-OC₂H₅), 4,3 (m, 2H, CH-OH), 6,1 - 6,7 (div. m, 10 olefinische H).

| Mikroanalyse (20 Mol% Methanol in den Kristallen): | | |
|---|---|---|
| Ber. | C 76,34% | H 9,36% |
| Gef. | C 75,92% | H 9,02% |

### Beispiel 16

### Herstellung von 3,3'-Dihydroxy-4,4'-diketo-β,β'-carotin, d.h. von Astaxanthin (Formel I, worin R¹ Hydroxy und m und n beide 1 bedeuten; "Durchprozess")

In einem mit Magnetrührer, Thermometer und Argonbegasung ausgestatteten 100 ml-Vierhalssulfierkolben wurden 60 ml Methylenchlorid vorgelegt und etwa 55 mg (6 Tropfen, 0,5 mmol) Acetondimethylacetal und etwa 25 mg (2 Tropfen, 0,2 mmol, 2 Mol%) Bortrifluorid-diethyletherat zugegeben. Nachdem diese Lösung etwa 16 Stunden bei Raumtemperatur stehengelassen wurde (zur Entfernung des Restwassers), kühlte man auf-25°C ab, gab 3,33 g (7,3 mmol) Crocetindialdehyd-diethylacetal (HPLC: 97,5%ig rein) und 3,80 g (21 mmol, 2,9 Aeq.) 4,6,6-Trimethyl-5,6,7,7a-tetrahydro-5-methyliden-1,3-benzodioxol in einer Portion und ohne Lösungsmittel zu. Nun wurde 9 Stunden bei -20°C bis -15°C unter HPLC-Kontrolle gerührt. Bei -20°C gab man nun 0,7 ml (etwa 1 g / 6 mmol) 48%ige wässrige Bromwasserstoffsäure zu und rührte 30 Minuten bei dieser Temperatur.

Zur Neutralisation gab man zum Gemisch in einem Schuss 10 ml (10 mmol) 1N-Natriumhydroxidlösung. Anschliessend rührte man im Eisbad 45 Minuten bei 0°C und säuerte dann mit 1 ml Essigsäure an. Dann wurde die Reaktionslösung mit Methylenchlorid extrahiert, zweimal mit Wasser gewaschen, über wasserfreiem Natriumsulfat getrocknet und eingeengt. Dies ergab 5,84 g dunkelroten, festen Rückstand, welchen man in 60 ml Heptan suspendierte. Die Suspension wurde 3 Stunden bei 100°C rückflussiert, langsam unter Rühren auf Raumtemperatur abgekühlt und filtriert. Die erhaltenen Kristalle wurden mit Heptan gewaschen. Nach Trocknung der Kristalle unter Hochvakuum bei Raumtemperatur erhielt man 4,10 g braunrotes Pulver. Dieses wurde durch Lösen in Methylenchlorid (60 ml bzw. 35 ml) und kontinuierliches Ersetzen durch Aceton (40 ml bzw. 30 ml) unter vermindertem Druck umkristallisiert. Abkühlen (-20°C) und Waschen mit kaltem (-20°C) Aceton ergab nach einstündiger Trocknung unter Hochvakuum bei 55°C 2,20 g (45%ige Ausbeute bezogen auf eingesetztes Crocetindialdehyd-diethylacetal) Astaxanthin als violette, glänzende Kristalle, Smp. 219°C; HPLC: 97% (Flächen-%).

Für die analytischen Daten wurde noch zweimal umkristallisiert. Dies ergab Astaxanthin als metallisch glänzende Kristalle, Smp. 219°C; HPLC: 97% (Flächen-%);

### HPLC (Gew.-% gegenüber Standard):

| | |
|---|---|
| (all E)-Astaxanthin | 94% |
| (9Z+13Z)-Astaxanthin | 0,3% |
| Mono-ethoxymethoxy-astaxanthin | 1,6% |
| 8'-Apoastaxanthinal | 1,6% |
| 3,3'-Dihydroxy-2,3-didehydro-4,4`-diketo-β,β'-carotin ("Halbastacin") | 0,1% |
| Methylenchlorid: | 2% |

UV (Cyclohexan/3% CHCl₃): 476 nm (log E = 5,10)

### Mikroanalyse (korrigiert mit 2% Methylenchlorid in den Kristallen):

| Mikroanalyse (korrigiert mit 2% Methylenchlorid in den Kristallen): | | |
|---|---|---|
| Ber. | C 78,95% | H 8,65% |
| Gef. | C 78,79% | H 8,52% |

### Beispiel 17

### Herstellung und Isolierung des Zwischenproduktes 4,8,4',8'-Tetramethoxy-7,8,7',8'-tetrahydro-3,4,3',4'-bis (O-methylen)-β,β'-carotin (Formel VI, worin R² Methyl, R⁵ und R⁶ beide Wasserstoff und m und n beide 1 bedeuten)

### (a) Katalyse durch Zinkchlorid, mit Acetonitril als Lösungsmittel

In einem mit Magnetrührer und Argonbegasung ausgestatteten 25 ml- Rundkolben wurden 580 mg (1,5 mmol) Crocetindialdehyd-dimethylacetal (HPLC: > 97%ig rein) und 815 mg (4,5 mmol) 4,6,6-Trimethyl-5,6,7,7a-tetrahydro-5-methyliden-1,3-benzodioxol in 10 ml Acetonitril vorgelegt und das Gemisch mit etwa 20 mg (10 Mol%) wasserfreiem Zinkchlorid bei 0°C versetzt. Die resultierende orange Suspension wurde nun 4 Tage bei -5°C und danach 5 Tage bei Raumtemperatur gerührt, wobei eine hellgelbe Suspension entstand. Nun wurde auf -10°C abgekühlt, abgenutscht und mit 8 ml Acetonitril bei -20°C gewaschen. Die gelben Kristalle wurden zur Reinigung in 10 ml heissem Methanol digeriert, auf -10°C abgekühlt, filtriert und unter Hochvakuum bei Raumtemperatur getrocknet. Dies ergab 690 mg (62%ige Ausbeute) 4,8,4',8'-Tetramethoxy-7,8,7'8'-tetrahydro-3,4,3',4'-bis (O-methylen)-β,β'-carotin als oranges Pulver.

Für die analytischen Daten wurde nochmals eine Probe analog aus Aceton digeriert. Dies ergab hellorange Kristalle, Smp. 170,5°C. UV (Cyclohexan/3% CHCl₃): 429 nm (logE = 5,14), 403 nm (logE = 5,12), 382 nm (logE = 4,91); IR (cm⁻¹): Keine C=O-Bande; Massenspektrum: 748,5 (M⁺, 5), 537,3 (50), 179 (100); ¹H-NMR (d₆-DMSO, 400 MHz; Diastereomerengemisch): u.a. 3,05, 3,17 (2s, OCH₃), 3,60, 3,70 (2m, C(3)-H), 4,98 und 5,07 (2m, OCH₂O), 6,10 - 6,70 (div. m, olefinische H).

### Mikroanalyse (mit 0,4% H₂O):

| Mikroanalyse (mit 0,4% H₂O): | | |
|---|---|---|
| Ber. | C 74,16% | H 8,66% |
| Gef. | C 74,07% | H 8,96% |

### (b) Katalyse durch Zinkdi(trifluorsulfonat), mit Acetonitril als Lösungsmittel

Zu 40 ml Acetonitril wurden 72 mg (etwa 8 Tropfen) Acetondimethylacetal gefolgt von 43 mg (0,12 mmol, 1,2 Mol%) Zinktriflat gegeben, und das Gemisch wurde etwa 16 Stunden gerührt. Dann gab man bei 0°C 4,01 g (10 mmol) Crocetindialdehyddimethylacetal (HPLC: ≥97%ig rein) und 4,60 g (25,6 mmol, 2,6 Aeq.) 4,6,6-Trimethyl-5,6,7,7a-tetrahydro-5-methyliden-1,3-benzodioxol zu und rührte 8 Stunden bei 0°C und 24 Stunden bei Raumtemperatur. Analog wie unter (a) beschrieben wurde nach Neutralisation mit etwa 0,2 ml Triethylamin auf -20°C abgekühlt und der gelbe dicke Brei abfiltriert und unter Hochvakuum bei Raumtemperatur getrocknet. Dies ergab 7,06 g (94%ige Ausbeute) 4,8,4',8'-Tetramethoxy-7,8,7',8'-tetrahydro-3,4,3',4'-bis(O-methylen)-β,β'-carotin als orange Kristalle.

### (c) Katalyse durch Eisen(III)chlorid, mit Methylenchlorid als Lösungsmittel

In einem mit Magnetrührer und Argonbegasung ausgestatteten 150 ml-Rundkolben wurden 100 mg (1 mmol) Aceton-dimethylacetal und 76 mg (0,5 mmol, 4 Mol%) wasserfreies Eisen(III)chlorid in 100 ml Methylenchlorid etwa 16 Stunden stehen gelassen. Dann wurde auf -30°C abgekühlt, und man gab 5,6 g (14,0 mmol) Crocetindialdehyddimethylacetal (HPLC:≥ 97%ig rein) gefolgt von 6,5 g (36 mmol) 4,6,6-Trimethyl-5,6,7,7a-tetrahydro-5-methyliden-1,3-benzodioxol ohne Lösungsmittel in einer Portion zu. Man rührte 7 Stunden bei -25 bis 30°C unter Kontrolle durch HPLC. Zur Neutralisation des Katalysators gab man 0,5 ml Triethylamin zu, ersetzte unter vermindertem Druck (150 mbar = 15 kPa) bei 40°C langsam das Methylenchlorid durch etwa 90 ml Methanol, kühlte das Gemisch auf -20°C ab, filtrierte die resultierenden Kristalle ab, wusch sie mit kaltem (-20°C) Methanol und trocknete sie unter Hochvakuum etwa 16 Stunden. Dies ergab 9,90 g (94%ige Ausbeute) 4,8,4',8'-Tetramethoxy-7,8,7',8'-tetrahydro-3,4,3',4'-bis(O-methylen)-β,β'-carotin als gelb-orange Kristalle, Smp. 169 - 172°C. Massenspektrum: 748,5 (M⁺, 2), 537,3 (10); ¹H-NMR (C₆D₆, 400 MHz; Diastereomerengemisch): u.a. 2,40, 2,75 (2m, 4H), 3,12 (s, OCH₃, 6H), - 3,35 (2s, OCH₃, 6H), 3,8 (m, 2H), 4,6 (m, 2H), 5,11 und 5,14 (2s, 2H), 5,33 (s, 2H), 6,3 - 6,8 (m, 10 olefinische H).

### (d) Katalyse durch Bortrifluor-diethyletherat, mit einem Gemisch von Methylenchlorid und Acetonitril als Lösungsmittel

Eine analoge Umsetzung [wie unter (b) beschrieben] von 2,72 g (6,8 mmol) Crocetindialdehyd-dimethylacetal (HPLC: ≥97%ig rein) mit 3,28 g (18,2 mmol) 4,6,6-Trimethyl-5,6,7,7a-tetrahydro-5-methyliden-1,3-benzodioxol in einem Gemisch von 50 ml Methylenchlorid und 10 ml Acetonitril mit etwa 40 mg (3 Tropfen, 0,3 mmol, 4 Mol%) Bortrifluorid-diethyletherat bei -30°C über 2 Stunden ergab nach Neutralisation mit Triethylamin und Kristallisation [analog (c)] 4,71 g (92%ige Ausbeute) 4,8,4',8'-Tetramethoxy-7,8,7',8'-tetrahydro-3,4,3',4'-bis(O-methylen)-β,β'-carotin als strohgelbe Kristalle, Smp. 175 - 177°C.

### Beispiel 18

### Herstellung von 3,3'-Dihydroxy-4,4'-diketo-β,β'-carotin, d.h. Astaxanthin (Formel I, worin R¹ Hydroxy und m und n beide 1 bedeuten)

### (a) Hydrolyse und Abspaltung in Methylenchlorid

In einem mit Magnetrührer und Argonbegasung ausgestatteten 100 ml-Rundkolben wurden 4,90 g (6,55 mmol) 4,8,4',8'-Tetramethoxy-7,8,7',8'-tetrahydro-3,4,3',4'-bis(O-methylen)-β,β'-carotin in 60 ml Methylenchlorid gelöst. Man kühlte die Lösung auf -20°C ab und versetzte sie mit 2 ml 48%iger wässriger Bromwasserstoffsäure, und zwar unter Kontrolle durch HPLC. Nach 1½ Stunden bei -15 bis -20°C wurde mit 20 ml 2N-Natriumhydroxidlösung neutralisiert und im Scheidetrichter abgetrennt. Die organische Phase wurde nun mit gesättigter Natriumbicarbonatlösung und danach mit halbgesättigter Natriumchloridlösung gewaschen, über wasserfreiem Natriumsulfat getrocknet, filtriert und auf etwa 35 ml eingeengt. Die Lösung wurde nun über ein Polster von 15 g Kieselgel [0,04 - 0,065 mm; Lösungsmittel:Methylenchlorid/Diethylether (3:1)] filtriert, unter vermindertem Druck etwas eingeengt und kontinuierlich durch Ethanol ersetzt, bis am Schluss etwa 20 ml Lösung blieb, wodurch Kristallisation eintrat. Die resultierende Suspension wurde mit einer kleinen Spatelspitze butyliertes Hydroxytoluen versetzt und zur Isomerisierung etwa 16 Stunden rückflussiert. Dann wurde auf 0°C abgekühlt, abgenutscht und mit kaltem Ethanol gewaschen. Dies ergab nach zweistündiger Trocknung bei 70°C unter Hochvakuum 3,00 g dunkelrote Kristalle, welche umkristallisiert wurden, und zwar durch Lösen in 50 ml Methylenchlorid, kontinuierliches Ersetzen des Lösungsmittels bei 40°C/400 mbar (40 kPa) durch Aceton (am Schluss etwa 30 ml Flüssigkeit), Abkühlen auf 0°C, Filtrieren und Waschen mit Aceton bei -20°C. Dies ergab nach 18-stündiger Trocknung bei Raumtemperatur unter Hochvakuum 2,80 g (68%ige Ausbeute, korr.) Astaxanthin als metallisch glänzende Kristalle, Smp. 219-220°C.

### HPLC-Gehalt (Gew.-% gegenüber Standard):

| | |
|---|---|
| (all E)-Astaxanthin | 94% |
| (9Z+13Z)-Astaxanthin | 0,7% |
| 8'-Apoastaxanthinal | 1,3% |
| Mono-methoxymethyl-astaxanthin | 1,6% |
| 3,3'-Dihydroxy-2,3-didehydro-4,4'-diketo-β,β'-carotin ("Halbastacin") | 0,3% |
| Methylenchlorid | 1% |

UV (Cydohexan/3% CHCl₃): 476 nm (logE = 5,10); Massenspektrum: 596 (M⁺, 10), 147 (100); ¹H-NMR (CDCl₃, 400 MHz): 1,21 und 1,32 (2s, je 6H), 1,81 (t, J = 12 Hz, 2H), 1,95, 1,99 und 2,00 (3s, 3 x 6H), 2,16 (d x d, J₁ = 12 Hz, J₂ = 6 Hz, 2 Hz), 3,68 (d, j ~ 2Hz, 2 x OH), 4,32 (d x d x d, J₁ = 12 Hz, J₂ = 6 Hz, J₃ ~ 2 Hz), 6,2 - 6,7 (div. m, 14 olefinische H).

### (b) Hydrolyse und Abspaltung in Acetonitril

In einem mit Magnetrührer und Argonbegasung ausgestatteten 100 ml-Rundkolben wurden 4,90 g (etwa 6,5 mmol) 4,8,4',8'-Tetramethoxy-7,8,7',8'-tetrahydro-3,4,3',4'-bis (O-methylen)-β,β'-carotin (Smp. 167 - 174°C) unter Argon vorgelegt. Unter Rühren wurden bei -15°C 2 ml (3 g, 20 mmol) 48%ige wässrige Bromwasserstoffsäure zu der gelben Suspension gegeben, wobei sich diese sofort dunkel färbte. Nach 15 Minuten wurde die Temperatur auf 0°C (Eisbad) erhöht und die Suspension bei dieser Temperatur 4 Stunden wietergerührt, und zwar unter HPLC-Kontrolle. Anschliessend wurde das Gemisch mit 20 ml (20 mmol) 1N-Natriumhydroxidlösung basisch gestellt und abgenutscht, und der Festkörper mit viel Wasser gewaschen. Die nassen Kristalle wurden nun in 250 ml Methylenchlorid gelöst, und die Lösung über wasserfreiem Natriumsulfat getrocknet, filtriert und unter vermindertem Druck auf etwa 50 ml eingeengt. Die konzentrierte Lösung wurde nun über ein Polster von 15 g Kieselgel (0,04 - 0,065 mm) unter Verwendung eines Lösungsmittelgemisches Methylenchlorid/Diethylether (3:1) filtriert und das Filtrat unter vermindertem Druck etwas eingeent. Man gab dann 60 ml Ethanol kontinuierlich zu und engte die Lösung weiter ein, bis das Schlussvolumen etwa 15 - 20 ml betrug. Die resultierende Suspension wurde nun zur Isomerisierung 2 Stunden am Rückfluss gekocht, auf Raumtemperatur abgekühlt und abfiltriert, und die Kristalle wurden mit etwa 10 ml Ethanol bei -20°C gewaschen. Zweistündige Trocknung bei 80°C unter Hochvakuum ergab 3,30 g Astaxanthin als dunkelviolette Kristalle, welche man aus 70 ml Methylenchlorid und 40 ml Aceton wie unter (a) beschrieben umkristallisierte. Dies ergab 3,10 g (74%ige Ausbeute) Astaxanthin als tiefviolette, glänzende Kristalle, Smp. 222°C; HPLC-Gehalt (Gew.% gegenüber Standard):

| | |
|---|---|
| (all E)-Astaxanthin | 91% |
| (9Z + 13Z)-Astaxanthin | 0,3% |
| 8'-Apoastaxanthinal | 1,7% |
| Mono-methoxymethyl-astaxanthin | 1,9% |
| 3,3'-Dihydroxy-2,3-didehydro-4,4'-diketo-β,β'-carotin ("Halbastacin") | 3,6% |
| Methylenchlorid | 1,4% |

UV(Cyclohexan/3% CHCl₃): 477 nm (logE = 5,07); Massenspektrum und ¹H-NMR identisch mit denjenigen eines authentischen Materials.

### Beispiel 19

### Herstellung von 3,3'-Dihydroxy-4,4'-diketo-β,β'-carotin, d.h. von Astaxanthin (Formel I, worin R¹ Hydroxy und m und n beide 1 bedeuten; "Durchprozess" II + III → I)

In einem mit Magnetrührer, Thermometer und Argonbegasung ausgestatteten 100 ml-Vierhalssulfierkolben wurden 50 ml Methylenchlorid vorgelegt und etwa 55 mg (6 Tropfen, 0,5 mmol) Acetondimethylacetal und 40 mg (3 Mol%) wasserfreies Eisen(III)chlorid zugegeben. Man rührte das Gemisch 2 Stunden bei 35 - 40°C. Nun kühlte man auf -30°C ab, gab 2,80 g (7 mmol) Crocetindialdehyd-dimethylacetal (HPLC: ≥97%ig rein) und 3,28 g (18,2 mmol, 2,6 Aeq.) 4,6,6-Trimethyl-5,6,7,7a-tetrahydro-5-methyliden-1,3-benzodioxol in einer Portion und ohne Lösungsmittel zu. Nun wurde bei -30°C bis -25°C 4½ Stunden gerührt, und zwar unter HPLC-Kontrolle. Bei -25°C gab man dann 1 ml (etwa 1,5 g / 9 mmol) 48%ige wässrige Bromwasserstoffsäure und 50 ml Methylenchlorid zu und rührte das Gemisch bei -15°C weitere 1¼ Stunden.

Zur Neutralisation gab man zum Gemisch in einem Schuss 5 ml (10 mmol) 2N-Natriumhydroxidlösung und rührte 5 Minuten. Dann wurde auf Wasser gegossen, das Wasser abgetrennt und die organische Phase mit gesättigter Natriumbicarbonat- und halbgesättigter Natriumchloridlösung gewaschen, über wasserfreiem Natriumsulfat getrocknet und unter vermindertem Druck auf etwa 30 ml eingeengt. Die konzentrierte Lösung wurde nun über ein Polster von 15 g Kieselgel (0,04 - 0,056 mm) unter Verwendung des Lösungsmittelgemisches Methylenchlorid/Diethylether (3:1) filtrierte, unter vermindertem Druck etwas eingeengt und kontinuierlich durch Ethanol ersetzt, bis das Schlussvolumen etwa 20 ml betrug, wodurch Kristallisation eintrat. Die resultierende Suspension wurde mit einer kleinen Spatelspitze butyliertes Hydroxytoluen versetzt und zur Isomerisierung etwa 16 Stunden rückflussiert. Dann wurde auf Raumtemperatur abgekühlt, abgenutscht und mit kaltem Ethanol gewaschen. Dies ergab nach zweistündiger Trocknung bei 70°C unter Hochvakuum 3,60 g violette Kristalle, welche man zweimal aus Methylenchlorid/Aceton wie unter Beispiel 18(a) beschrieben umkristallisierte. Auf diese Weise erhielt man 3,00 g (68%ige Ausbeute bezogen auf eingesetztes Crocetindialdehyddimethylacetal) Astaxanthin als tiefviolette, glänzende Kristalle, Smp. 219°C. HPLC-Gehalt (Gew.% gegenüber Standard):

| | |
|---|---|
| (all E)-Astaxanthin | 95% |
| (9Z+13Z)-Astaxanthin | 0,2% |
| Mono-methoxymethyl-astaxanthin: | 1% |
| 8'-Apoastaxanthinal | 1% |
| 3,3'-Dihydroxy-2,3-didehydro-4,4'-diketo-β,'-carotin ("Halbastacin") | 0,3% |
| Methylenchlorid | 1% |

UV (Cyclohexan/3% CHCl₃): 476 nm (log E = 5,10); Massenspektrum und ¹H-NMR- Spektrum identisch mit denjenigen eines authentischen Materials.

## Patentansprüche

1. Verfahren zur Herstellung eines symmetrischen, endständig ringsubstituierten Polyens der allgemeinen Formel 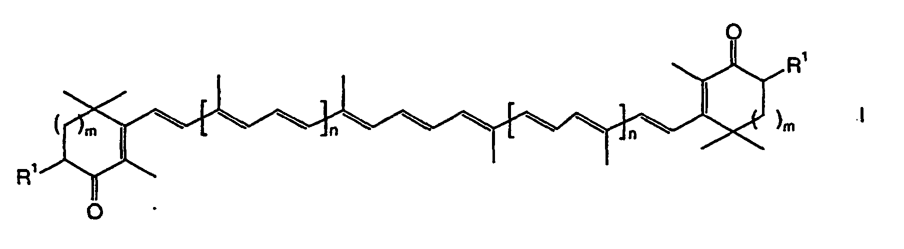 worin
R¹ Wasserstoff oder Hydroxy,
m 0 oder 1 und
n 0, 1 oder 2 bedeuten,
**dadurch gekennzeichnet, dass** man ein Polyen-di(O,O-dialkylacetal) der allgemeinen Formel 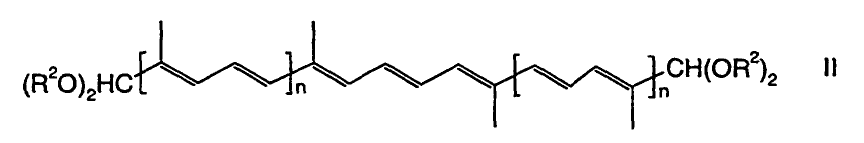 worin
R² C₁₋₆-Alkyl bedeutet und
n die oben angegebene Bedeutung besitzt,
mit einem cyclischen Dienolether der allgemeinen Formel 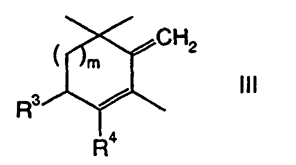 worin
R³ Wasserstoff und
R⁴ C₁₋₄-Alkoxy, oder
R³ und R⁴zusammen eine gegebenenfalls substituierte Methylendioxygruppe - O-C(R⁵)(R⁶)-O-, und
R⁵ und R⁶unabhängig voneinander Wasserstoff, C₁₋₄-Alkyl oder Phenyl bedeuten und
m die oben angegebene Bedeutung besitzt,
in Gegenwart einer Lewis- oder Brönstedsäure umsetzt, das Reaktionsprodukt unter sauren Bedingungen hydrolysiert, und von der so erhaltenen Verbindung der allgemeinen Formel 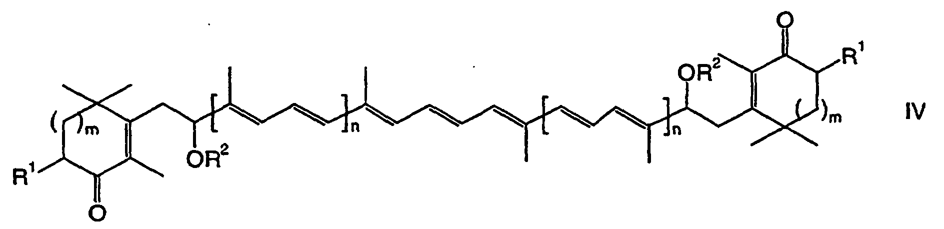 worin
R¹ Wasserstoff oder Hydroxy bedeutet, je nachdem ob R³ und R⁴ der Formel III Wasserstoff resp. C₁₋₄-Alkoxy bzw. zusammen die gebenenfalls substituierte Methylendioxygruppe bedeuten, und
R², m und n die oben angegebenen Bedeutungen besitzen,
unter basischen oder sauren Bedingungen das Alkanol R²OH abspaltet.

2. Verfahren nach Anspruch 1, **dadurch gekennzeichnet, dass** R² Methyl, entweder R³ Wasserstoff und R⁴ Isobutoxy oder R³ und R⁴ zusammen die Methylendioxygruppe, und n 1 bedeuten.

3. Verfahren nach Anspruch 1 oder 2, **dadurch gekennzeichnet, dass** als Lewissäure Zinkchlorid, Zinkchloriddietherat, Zinkbromid, Zinkdi(trifluormethansulfonat), Titantetrachlorid, Zinntetrachlorid, Bortrifluoridetherat oder Eisen(III)chlorid, und als Brönstedsäure p-Toluensulfonsäure, Methansulfonsäure, Trifluormethansulfonsäure, Schwefelsäure oder Trifluoressigsäure verwendet wird.

4. Verfahren nach Anspruch 3, **dadurch gekennzeichnet, dass** als Lewis- oder Brönstedsäure eins der genannten Zinksalze, Bortrifluoridetherat oder Eisen(III)chlorid verwendet wird.

5. Verfahren nach einem der Ansprüche 1 bis 4, **dadurch gekennzeichnet, dass** die Lewis- bzw. Brönstedsäure in katalytischer Menge eingesetzt wird, welche etwa 0,5 bis etwa 30 Molprozent, vorzugsweise etwa 5 bis etwa 10 Molprozent, bezogen auf die eingesetzte Menge des Polyen-di(O,O-dialkylacetals) der Formel II beträgt

6. Verfahren nach einem der Ansprüche 1 bis 5, **dadurch gekennzeichnet, dass** man etwa 2,1 bis etwa 4 Aequivalente, vorzugsweise etwa 2,2 bis etwa 2,6 Aequivalente, cyclischen Dienolether der Formel III pro Aequivalent Polyen-di(O,O-dialkylacetal) der Formel II verwendet.

7. Verfahren nach einem der Ansprüche 1 bis 6, **dadurch gekennzeichnet, dass** man das Polyen-di(O,O-dialkylacetal) der Formel II mit dem cyclischen Dienolether der Formel III in einem organischen Lösungsmittel bei Temperaturen im Bereich von etwa -50°C bis etwa +60°C, vorzugsweise im Temperaturbereich von etwa -30°C bis zur Raumtemperatur, umsetzt, wobei als organisches Lösungsmittel ein niederer halogenierter aliphatischer Kohlenwasserstoff, z.B. Methylenchlorid oder Chloroform; ein niedere aliphatischer oder cyclischer Ether, z.B. Diethylether, tert.Butylmethylether oder Tetrahydrofuran; ein niederes aliphatisches Nitril, z.B. Acetonitril; ein niederer aliphatischer Ester, z.B. Ethylacetat; oder ein aromatischer Kohlenwasserstoff, z.B. Toluen, verwendet wird.

8. Verfahren nach Anspruch 7, **dadurch gekennzeichnet, dass** der Temperaturbereich etwa -25°C bis etwa +60°C, vorzugsweise etwa 0°C bis zur Raumtemperatur, beträgt.

9. Verfahren nach Anspruch 7 oder 8, **dadurch gekennzeichnet, dass** als organisches Lösungsmittel ein Gemisch von Acetonitril mit Ethylacetat oder Methylenchlorid verwendet wird, vorzugsweise eins, in welchem das Volumenverhältnis Acetonitril: Ethylacetat bzw Methylenchlorid etwa 1:1 bis etwa 1:4 beträgt.

10. Verfahren nach Anspruch 9, **dadurch gekennzeichnet, dass** als organisches Lösungsmittel ein Gemisch von Acetonitril mit Ethylacetat verwendet wird.

11. Verfahren nach einem der Ansprüche 1 bis 10, **dadurch gekennzeichnet, dass** man unmittelbar nach Beendigung der Reaktion des Polyen-di(O,O-dialkylacetals) der Formel II mit dem cyclischen Dienolether der Formel III das daraus resultierende Zwischenprodukt im Reaktionsgemisch selber hydrolysiert, indem man zum Reaktionsgemisch eine Säure, vorzugsweise leicht verdünnte wässrige Essigsäure, gibt und das Gemisch anschliessend im Temperaturbereich von etwa 0°C bis etwa 50°C rührt.

12. Verbindungen der allgemeinen Formel 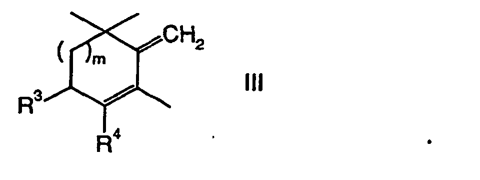 worin
R³ Wasserstoff und
R⁴ C₁₋₄-Alkoxy, oder
R³ und R⁴ zusammen eine gegebenenfalls substituierte Methylendioxygruppe - O-C(R⁵)(R⁶)-O- und
R⁵ und R⁶ unabhängig voneinander Wasserstoff, C₁₋₄-Alkyl oder Phenyl, und
m 0 oder 1 bedeuten.

13. Verbindungen der allgemeinen Formel 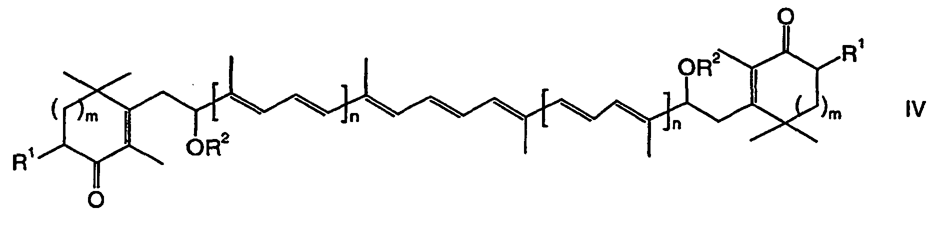 worin
R¹ Wasserstoff oder Hydroxy,
R² C₁₋₆-Alkyl,
m 0 oder 1 und
n 0, 1 oder 2 bedeuten.

14. Verbindungen der allgemeinen Formel 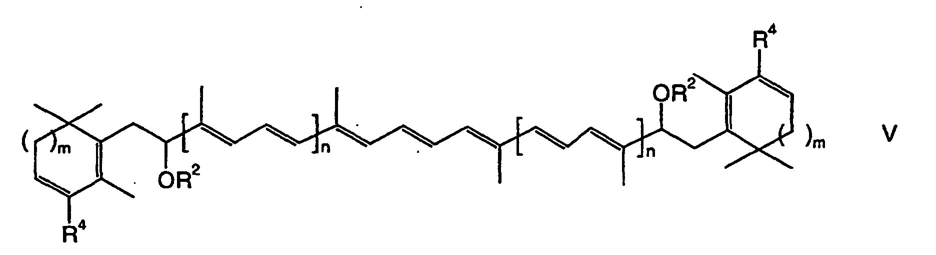 worin
R² C₁₋₆-Alkyl,
R⁴ C₁₋₄-Alkoxy
m 0 oder 1 und
n 0, 1 oder 2 bedeuten.

15. Verbindungen der allgemeinen Formel 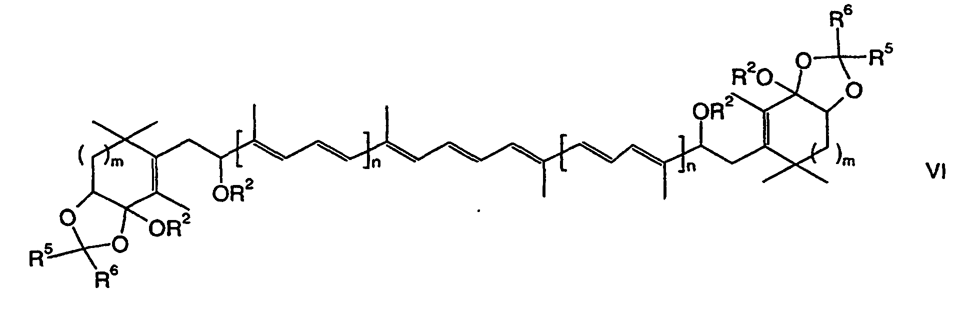 worin
R² C₁₋₆-Alkyl,
R⁵ und R⁶ unabhängig voneinander Wasserstoff, C₁₋₄-Alkyl oder Phenyl,
m 0 oder 1 und
n 0, 1 oder 2 bedeuten.

## Claims

1. A process for the manufacture of a symmetrical, terminally ring-substituted polyene of formula 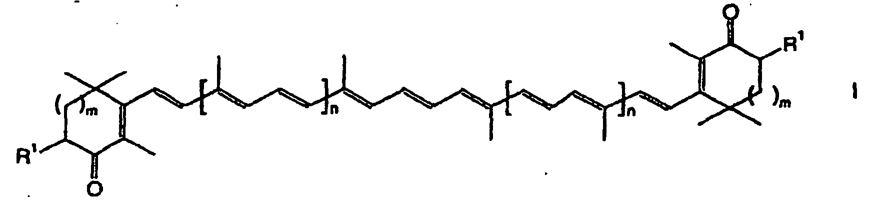 wherein
R¹ is hydrogen or hydroxy,
m is 0 or 1 and
n is 0, 1 or 2,
which comprises
reacting a polyene di(O,O-dialkyl acetal) of formula 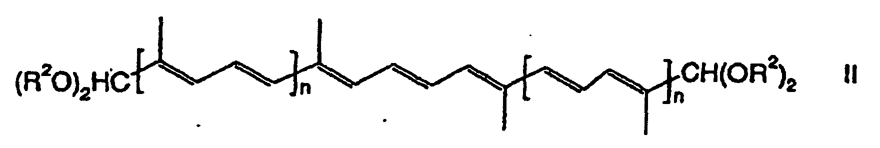 wherein
R² is C₁₋₆-alkyl and
n is as defined above,
with a cyclic dienol ether of formula 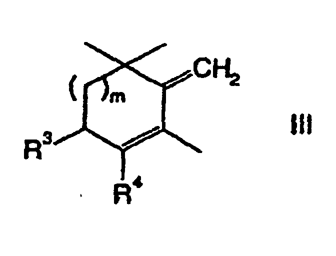 wherein
R³ is hydrogen and
R⁴ is C₁₋₄-alkoxy, or
R³ and R⁴ together form an optionally substituted methylenedioxy group, -O-C(R⁵)(R⁶)-O-, and
R⁵ and R⁶ are each independently hydrogen, C₁₋₄-alkyl or phenyl, and
m is as defined above,
in the presence of a Lewis or Brönsted acid,
hydrolyzing the reaction product under acidic conditions and cleaving off the alkanol R²OH from the thus-obtained compound of formula 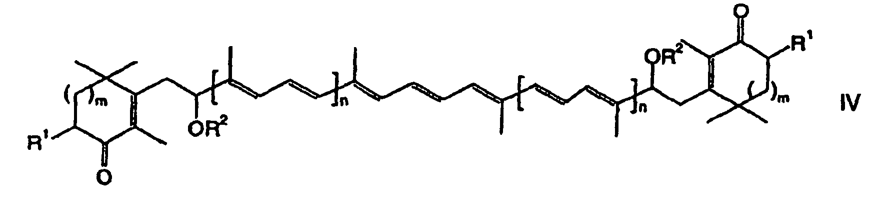 wherein
R¹ is hydrogen or hydroxy, depending on whether R³ and
R⁴ in formula III are hydrogen and C₁₋₄-alkoxy, respectively, or together signify the optionally substituted methylenedioxy group, and
R², m and n are as defined above,
under basic or acidic conditions.

2. The process of claim 1, wherein R² is methyl, R³ is hydrogen and R⁴ is isobutoxy, or R³ and R⁴ together form the methylenedioxy group, and n is 1.

3. The process of claim 1 or 2, wherein the Lewis acid is selected from the group consisting of zinc chloride, zinc chloride dietherate, zinc bromide, zinc di(trifluoromethanesulphonate), titanium tetrachloride, tin tetrachloride, boron trifluoride etherate and iron(III) chloride and the Brönsted acid is selected from the group consisting of p-toluenesulphonic acid, methanesulphonic acid, trifluoromethanesulphonic acid, sulphuric acid and trifluoroacetic acid.

4. The process of claim 3, wherein the Lewis or Brönsted acid used is one of the zinc salts mentioned, boron trifluoride etherate or iron(III) chloride.

5. The process of any of claims 1 to 4, wherein the Lewis or Brönsted acid is used in a catalytic amount, which is about 0.5 to about 30 mol percent, preferably about 5 to about 10 mol percent, based on the amount of polyene di(O,O-dialkyl acetal) of formula II used.

6. The process of any of claims 1 to 5, wherein about 2.1 to about 4 equivalents, preferably about 2.2 to about 2.6 equivalents, of cyclic dienol ether of formula III are used per equivalent of polyene di (O, O-dialkyl acetal) of formula II.

7. The process of any of claims 1 to 6, wherein the polyene-di(0,0-dialkyl acetal) of formula II is reacted with the cyclic dienol ether of formula III in an organic solvent at temperatures in the range of about -50°C to about +60°C, preferably in the temperature range of about -30°C to room temperature, using as organic solvent a lower halogenated aliphatic hydrocarbon, e.g., methylene chloride or chloroform; a lower aliphatic or cyclic ether, e.g., diethyl ether, tert-butyl methyl ether or tetrahydrofuran; a lower aliphatic nitrile, e.g., acetonitrile; a lower aliphatic ester, e.g., ethyl acetate; or an aromatic hydrocarbon, e.g., toluene.

8. The process of claim 7, wherein the temperature range is about -25°C to about +60°C, preferably about 0°C to room temperature.

9. The process of claim 7 or 8, wherein a mixture of acetonitrile with ethyl acetate or methylene chloride, preferably one in which the acetonitrile: ethyl acetate or methylene chloride volume ratio is about 1:1 to about 1:4, is used as the organic solvent.

10. The process of claim 9, wherein a mixture of acetonitrile with ethyl acetate is used as the organic solvent.

11. The process of any of claims 1 to 10, wherein immediately after completion of the reaction of the polyene di(O,O-dialkyl acetal) of formula II with the cyclic dienol ether of formula III, the intermediate resulting therefrom is itself hydrolyzed in the reaction mixture by addition of an acid, preferably slightly diluted aqueous acetic acid, to the reaction mixture and subsequently stirring of the mixture at a temperature in the range of about 0°C to about 50°C.

12. Compounds of general formula 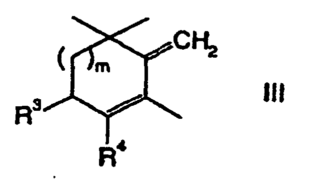 wherein
R³ is hydrogen and
R⁴ is C₁₋₄-alkoxy, or
R³ and R⁴ together form an optionally substituted methylenedioxy group -O-C(R⁵)(R⁶)-O-, and
R⁵ and R⁶ are each independently hydrogen, C₁₋₄-alkyl or phenyl, and
m is 0 or 1.

13. Compounds of general formula 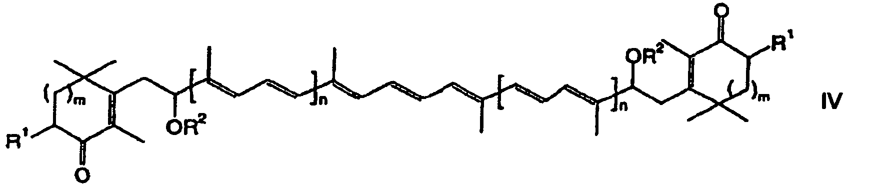 wherein
R¹ is hydrogen or hydroxy,
R² is C₁₋₆-alkyl,
m is 0 or 1, and
n is 0, 1 or 2.

14. Compounds of general formula 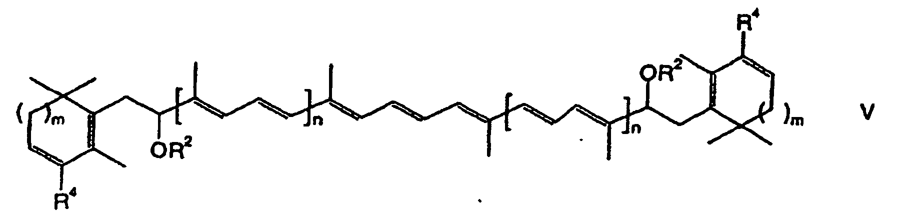 wherein
R² is C₁₋₆-alkyl,
R⁴ is C₁₋₄-alkoxy,
m is 0 or 1, and
n is 0, 1 or 2.

15. Compounds of general formula 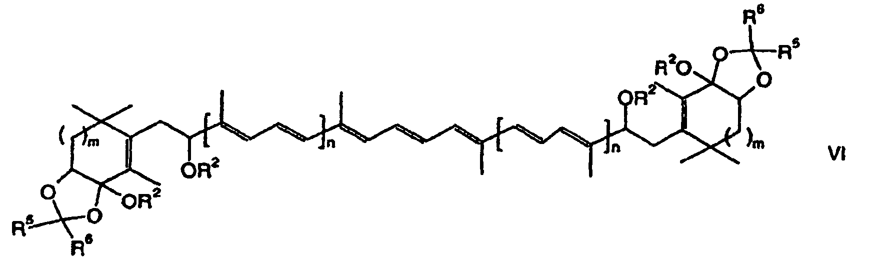 wherein
R² is C₁₋₆-alkyl,
R⁵ and R⁶ are each independently hydrogen, C₁₋₄-alkyl or phenyl,
m is 0 or 1, and
n is 0, 1 or 2.

## Revendications

1. Procédé pour la préparation d'un polyène symétrique, substitué sur le cycle en bout de chaîne, de formule générale 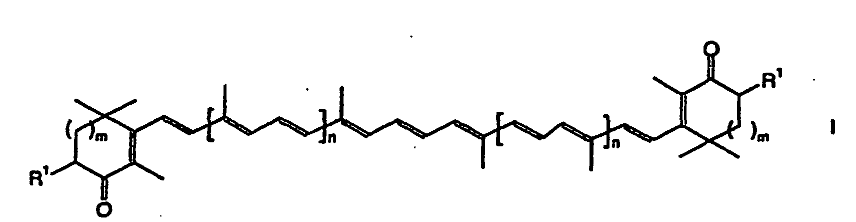 dans laquelle
R¹ représente un hydrogène ou hydroxy,
m représente 0 ou 1 et
n représente 0, 1 ou 2,
**caractérisé en ce que** l'on fait réagir un polyène-di(O,O-dialkylacétal) de formule générale 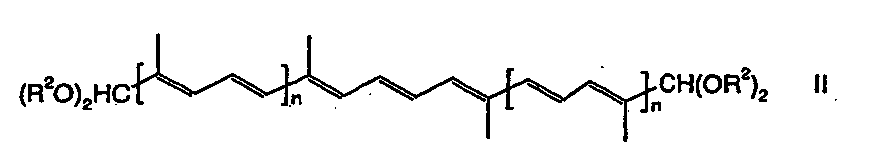 dans laquelle
R² représente un alkyle en C₁ à C₆ et
n possède la signification indiquée ci-dessus,
avec un diénoléther cyclique de formule générale 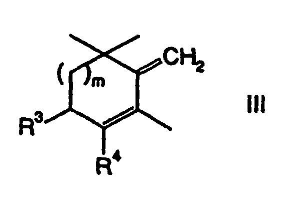 dans laquelle
R³ représente un hydrogène et
R⁴ représente un alcoxy en C₁ à C₄, ou
R³ et R⁴ représentent ensemble un groupe méthylènedioxy -O-C(R⁵) (R⁶) -O- éventuel lement substitué, et
R⁵ et R⁶ représentent indépendamment l'un de l'autre un hydrogène, un alkyle en C₁ à C₄ ou un phényle et
m possède la signification indiquée ci- dessus,
en présence d'un acide de Lewis ou de Brönsted, on hydrolyse le produit de réaction dans des conditions acides, et du composé ainsi obtenu de formule générale 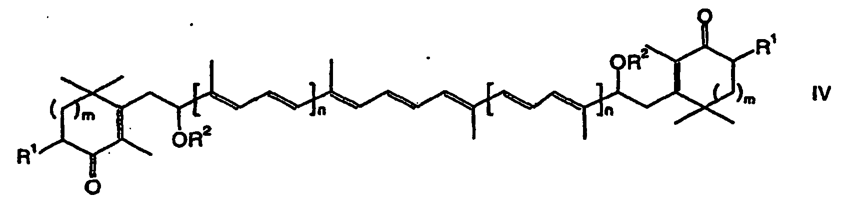 dans laquelle
R¹ représente un hydrogène ou hydroxy, selon si R³ et R⁴ de la formule III représentent un hydrogène resp. un alcoxy en C₁ à C₄ ou représentent ensemble le groupe méthylènedioxy éventuellement substitué, et
R², m et n possèdent les significations indiquées ci-dessus,
on élimine l'alcanol R²OH dans des conditions basiques ou acides.

2. Procédé selon la revendication 1, **caractérisé en ce que** R² représente un méthyle, soit R³ représente un hydrogène et R⁴ un isobutoxy soit R³ et R⁴ représentent ensemble le groupe méthylènedioxy et n est 1.

3. Procédé selon la revendication 1 ou 2, **caractérisé en ce qu'**on utilise comme acide de Lewis le chlorure de zinc, le diéthérate de chlorure de zinc, le bromure de zinc, le di(trifluorométhanesulfonate) de zinc, le tétrachlorure de titane, le tétrachlorure d'étain, le diéthérate de trifluorure de bore ou le chlorure de fer(III), et comme acide de Brônsted l'acide p-toluènesulfonique, l'acide méthanesulfonique, l'acide trifluorométhanesulfonique, l'acide sulfurique ou l'acide trifluoroacétique.

4. Procédé selon la revendication 3, **caractérisé en ce qu'**on utilise comme acide de Lewis ou de Brônsted l'un des sels de zinc cités, l'éthérate de trifluorure de bore ou le chlorure de fer (III).

5. Procédé selon l'une des revendications 1 à 4, **caractérisé en ce que** l'on utilise l'acide de Lewis ou de Brônsted en quantité catalytique, qui s'élève d'environ 0,5 à environ 30 pour cent en moles, de préférence d'environ 5 à environ 10 pour cent en moles, par rapport à la quantité mise en oeuvre de polyène-di(O,O-dialkylacétal) de formule II.

6. Procédé selon l'une des revendications 1 à 5, **caractérisé en ce qu'**on utilise environ 2,1 à environ 4 équivalents, de préférence environ 2,2 à environ 2,6 équivalents, de diénoéthers cycliques de formule III par équivalent de polyène-di(O,O-dialkyacétal) de formule II.

7. Procédé selon l'une des revendications 1 à 6, **caractérisé en ce que** l'on fait réagir le polyène-di(O,O-dialkylacétal) de formule II avec le diénoléther cyclique de formule III dans un solvant organique à des températures dans la gamme d'environ -50 °C à environ + 60 °C, de préférence dans la gamme de température d'environ -30 °C à la température ambiante, dans lequel on utilise comme solvant organique un hydrocarbure aliphatique halogéné inférieur, par exemple le chlorure de méthylène ou le chloroforme ; un éther aliphatique ou cyclique inférieur, par exemple le diéthyléther, le tert.-butyl-méthyléther ou le tétrahydrofurane ; un nitrile aliphatique inférieur, par exemple l'acétonitrile ; un ester aliphatique inférieur, par exemple l'acétate d'éthyle ; ou un hydrocarbure aromatique, par exemple le toluène.

8. Procédé selon la revendication 7, **caractérisé en ce que** la gamme de température est d'environ -25 °C à environ + 60 °C, de préférence d'environ 0 °C à la température ambiante.

9. Procédé selon la revendication 7 ou 8, **caractérisé en ce qu'**on utilise comme solvant organique un mélange d'acétonitrile avec l'acétate d'éthyle ou le chlorure de méthylène, de préférence un solvant dans lequel le rapport en volume acétonitrile : acétate d'éthyle ou chlorure de méthylène est d'environ 1 : 1 à environ 1 : 4.

10. Procédé selon la revendication 9, **caractérisé en ce qu'**on utilise comme solvant organique un mélange d'acétonitrile avec l'acétate d'éthyle.

11. Procédé selon l'une des revendications 1 à 10, **caractérisé en ce qu'**immédiatement après la fin de la réaction du polyène-di(O,O-dialkylacétal) de formule II avec le diénoléther cyclique de formule III le produit intermédiaire en résultant s'hydrolyse de lui-même dans le mélange réactionnel en ajoutant au mélange réactionnel un acide, de préférence l'acide acétique aqueux légèrement dilué et en agitant ensuite le mélange dans la gamme de températures d'environ 0 °C à environ 50 °C.

12. Composés de formule générale 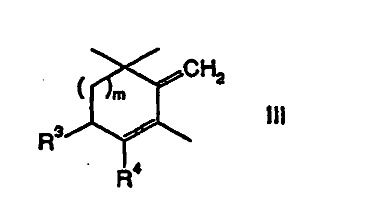 dans laquelle
R³ représente un hydrogène et
R⁴ représente un alcoxy en C₁ à C₄, ou
R³ et R⁴ représentent ensemble un groupe méthylènedioxy -O-C(R⁵) (R⁶) -O- éventuel lement substitué et
R⁵ et R⁶ représentent indépendamment l'un de l'autre un hydrogène, un alkyle en C₁ à C₄ ou un phényle, et
m représente 0 ou 1.

13. Composés de formule générale 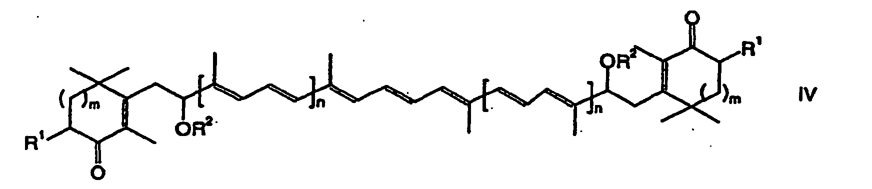 dans laquelle
R¹ représente un hydrogène ou hydroxy,
R² représente un alkyle en C₁ à C₆,
m représente 0 ou 1,
n représente 0, 1 ou 2.

14. Composés de formule générale 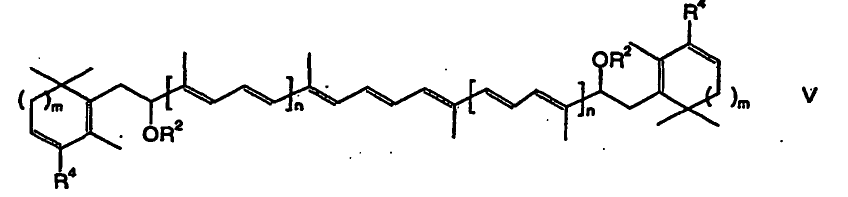 dans laquelle
R² représente un alkyle en C₁ à C₆,
R⁴ représente un alcoxy en C₁ à C₄,
m représente 0 ou 1, et
n représente 0, 1 ou 2.

15. Composés de formule générale 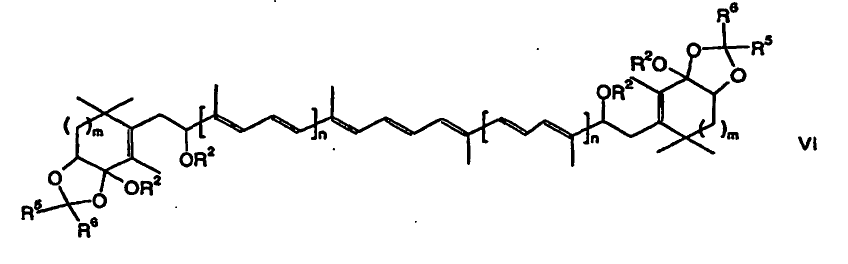 dans laquelle
R² représente un alkyle en C₁ à C₆,
R⁵ et R⁶ représentent indépendamment l'un de l'autre un hydrogène, un alkyle en C₁ à C₄, ou un phényle,
m représente 0 ou 1, et
n représente 0, 1 ou 2.
